(19) Europäisches Patentamt
European Patent Office
Office européen des brevets

(11) EP 4 640 673 A1

(12) **EUROPEAN PATENT APPLICATION**
published in accordance with Art. 153(4) EPC

(43) Date of publication:
**29.10.2025 Bulletin 2025/44**

(21) Application number: **23906090.8**

(22) Date of filing: **22.12.2023**

(51) International Patent Classification (IPC):
***C07D 237/24*** (2006.01) ***C07D 403/12*** (2006.01)
***C07D 401/12*** (2006.01) ***C07D 213/82*** (2006.01)
***C07B 59/00*** (2006.01) ***A61K 31/50*** (2006.01)
***A61K 31/501*** (2006.01) ***A61K 31/506*** (2006.01)
***A61K 31/44*** (2006.01) ***A61K 31/4439*** (2006.01)
***A61K 31/444*** (2006.01) ***A61P 17/06*** (2006.01)
***A61P 19/02*** (2006.01) ***A61P 17/00*** (2006.01)
***A61P 1/00*** (2006.01) ***A61P 29/00*** (2006.01)
***A61P 27/02*** (2006.01)

(52) Cooperative Patent Classification (CPC):
**A61K 31/44; A61K 31/4439; A61K 31/444; A61K 31/50; A61K 31/501; A61K 31/506; A61P 1/00; A61P 17/00; A61P 17/06; A61P 19/02; A61P 27/02; A61P 29/00; C07B 59/00; C07D 213/82; C07D 237/24;** (Cont.)

(86) International application number:
**PCT/CN2023/140951**

(87) International publication number:
**WO 2024/131935 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2022 CN 202211669072**

(71) Applicant: **Tianjin Pharmaceutical Biotechnology Co., Ltd**
**Tianjin 300171 (CN)**

(72) Inventors:
- **WANG, Wenzhi**
  **Tianjin 300171 (CN)**
- **SUN, Shuangyong**
  **Tianjin 300171 (CN)**
- **YAO, Chenhui**
  **Tianjin 300171 (CN)**
- **AN, Jiali**
  **Tianjin 300171 (CN)**
- **ZHANG, Jie**
  **Tianjin 300171 (CN)**
- **LIU, Dengke**
  **Tianjin 300171 (CN)**
- **HAN, Kunying**
  **Tianjin 300171 (CN)**
- **WANG, Ruina**
  **Tianjin 300171 (CN)**

(74) Representative: **Burger, Hannes Alfred**
**Anwälte Burger & Partner**
**Rechtsanwalt GmbH**
**Rosenauerweg 16**
**4580 Windischgarsten (AT)**

# (54) NOVEL AROMATIC RING DERIVATIVE CONTAINING AMIDE SUBSTITUTION AND USE THEREOF

(57) The present invention belongs to the technical field of medicine, and in particular relates to a novel aromatic ring derivative containing an amide substitution or a pharmaceutically acceptable salt thereof. The diseases comprise, but are not limited to, inflammatory or autoimmune diseases such as psoriasis, psoriatic arthritis, dermatitis, lupus erythematosus, inflammatory bowel disease, hidradenitis suppurativa, rheumatoid arthritis, and uveitis. The structure of the compound of formula I is as described in the claims and the description. The aromatic ring derivative containing an amide substitution provided in the present invention is used as a novel

EP 4 640 673 A1

TYK2 inhibitor, has a good TYK2 inhibitory effect, has a good therapeutic effect in an in vivo model of psoriasis and asthma animals, and has good druggability, high stability and good safety.

(52) Cooperative Patent Classification (CPC): (Cont.)
**C07D 401/12; C07D 403/12**

## Description

## Technical field

The present invention belongs to the technical field of medicinal chemistry, and in particular, relates to a novel aromatic ring derivative containing an amide substitution and use thereof for the manufacture of a medicament for the treatment of diseases, including but not limited to inflammatory or autoimmune diseases such as psoriasis, psoriatic arthritis, dermatitis, lupus erythematosus, inflammatory bowel disease, hidradenitis suppurativa, rheumatoid arthritis, and nephritis.

## Background art

Janus kinases (JAKs) are a family of intracellular non-receptor tyrosine kinases that mediate the signaling of most cytokines in cells, including interleukins (ILs), interferons (IFNs), erythropoietin (EPO), granulocyte macrophage-colony stimulating factor (GM-CSF), somatotropin (GH), prolactin (PRL), thrombopoietin (TPO), platelet-derived growth factor (PDGF), and epidermal growth factor (EGF) and the like. Since JAK family members can mediate the signal pathways of various cytokines, currently approved JAK inhibitors (targeting JAK 1-3) inevitably bring about some side effects, and a black box warning is added to the label of most JAK inhibitors approved by the FDA, which greatly limits their clinical application range. For example, early JAK inhibitors such as Tofacitinib have a better curative effect, but they all have higher inhibitory activity on JAK-1, JAK-2, and JAK-3 and greater side effects. It is required by the FDA to add the risk of thrombosis and death with a black box warning in the Tofacitinib instructions. While other JAK inhibitors, such as upadacitinib and baricitinib, all have a risk of "serious infection, malignancy, and thrombosis". TYK2 is a member of the JAK family and plays a crucial role in regulating the signaling cascade downstream of IL-12, IL-23, and type I interferon receptors. IL-12 and IL-23 are currently considered key cytokines that affect the progression of psoriasis disease. In addition, TYK2-mediated signaling is also associated with various inflammatory, and autoimmune diseases such as arthritis, dermatitis, lupus erythematosus and inflammatory bowel disease. Therefore, there is a significant need for novel TYK2 inhibitors that offer both high efficacy and improved safety.

## Contents of the invention

The present invention provides the following technical solution:
In a first aspect, Embodiment 1: a novel aromatic ring derivative containing an amide substitution or a pharmaceutically acceptable salt thereof is provided, characterized in that the compound is expressed by formula I:

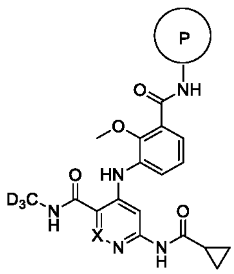

(I)

wherein, in the compound of formula I, X and P are selected independently from each other, and:

X is selected from CH, N;

P is selected from C1-C6 alkyl, substituted or unsubstituted C6-C10 aryl, substituted or unsubstituted five-membered heteroaryl, substituted or unsubstituted six-membered heteroaryl, wherein the substituent is selected from one or more of: C1-C6 alkyl, C3-C6 cycloalkyl, halogen, C1-C6 alkoxy, C1-C6 haloalkyl, -CN;

when X is N, P is as defined above; or

when X is CH, P is as defined above.

Embodiment 2: the compound of formula I or a pharmaceutically acceptable salt thereof as described in Embodiment 1, characterized in that, in the compound of formula I, X and P are selected independently from each other, and:

X is selected from CH, N;
P is selected from C1-C4 alkyl, substituted or unsubstituted phenyl, substituted or unsubstituted five-membered heteroaryl containing 2 to 3 N, substituted or unsubstituted six-membered heteroaryl containing 1 to 2 N, wherein the substituent is selected from 1 or 2 of: C1-C4 alkyl, C3-C6 cycloalkyl, halogen, C1-C4 alkoxy, C1-C4 haloalkyl, -CN;
when X is N, P is as defined above; or
when X is CH, P is as defined above.

Embodiment 3: the compound of formula I or a pharmaceutically acceptable salt thereof as described in Embodiment 1, characterized in that, in the compound of formula I, X and P are selected independently from each other, and:

X is selected from CH, N;
P is selected from tert-butyl, substituted or unsubstituted phenyl, substituted or unsubstituted five-membered heteroaryl selected from

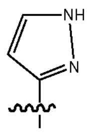 , 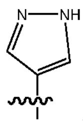 , 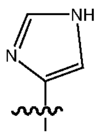 and 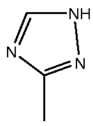 ,

and substituted or unsubstituted six-membered heteroaryl selected from

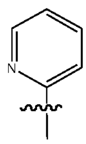 , 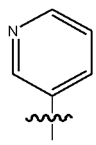 ,

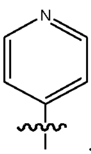 , 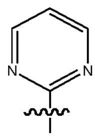 and 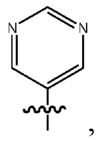 ,

wherein the substituent is selected from 1 or 2 of: methyl, ethyl, isopropyl, tert-butyl, cyclopropyl, methoxy, cyano, fluoro and trifluoromethyl;
when X is N, P is as defined above; or
when X is CH, P is as defined above.

Embodiment 4: the compound of formula I or a pharmaceutically acceptable salt thereof as described in Embodiment 1, characterized in that, in the compound of formula I, X and P are selected independently from each other, and:

X is selected from CH, N;

P is selected from tert-butyl, substituted or unsubstituted phenyl, substituted or unsubstituted five-membered heteroaryl selected from

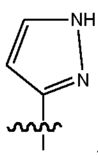 , 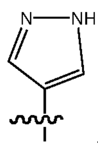 , 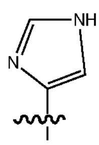 and 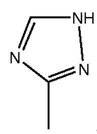 ,

and substituted or unsubstituted six-membered heteroaryl selected from

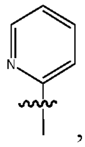, 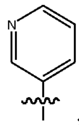, 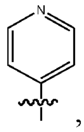,

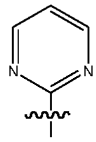 and 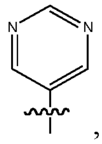,

for the above phenyl, the substituent is selected from 1 or 2 of: methyl, ethyl, isopropyl, tert-butyl, cyclopropyl, methoxy, cyano, fluoro and trifluoromethyl;

for the above five-membered heteroaryl, the substituent is methyl;

for the above six-membered heteroaryl, the substituent is selected from methyl or fluoro;

when X is N, P is as defined above; or

when X is CH, P is as defined above.

Embodiment 5: the compound of formula I or a pharmaceutically acceptable salt thereof as described in Embodiment 1, characterized in that, in the compound of formula I, X and P are selected independently from each other, and:

X is selected from CH, N;
P is selected from tert-butyl,

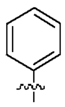, 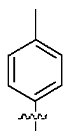, 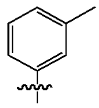, 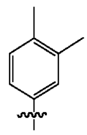, 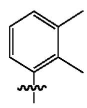, 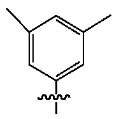, 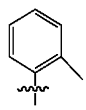,

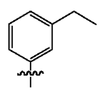, 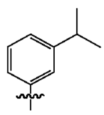, 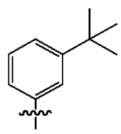, 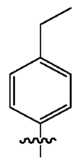, 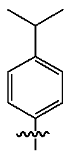, 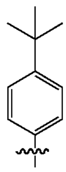, 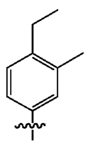, 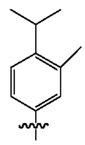, 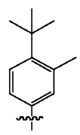, 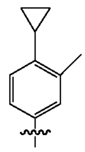, 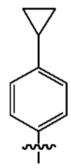,

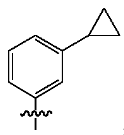, 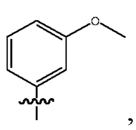, 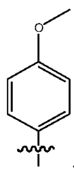, 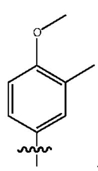, 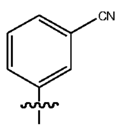, 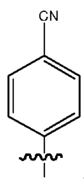, 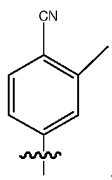, 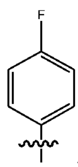, 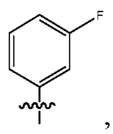, 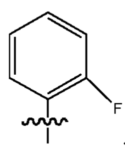,

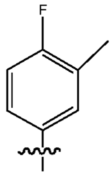, 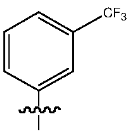, 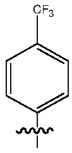, 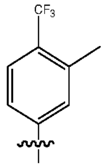, 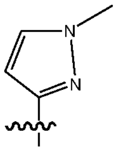, 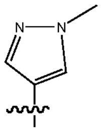, 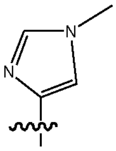, 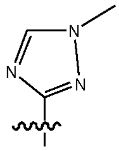, 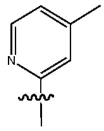,

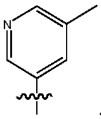, 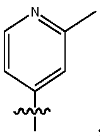, 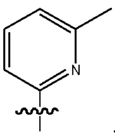, 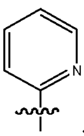, 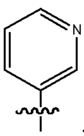, 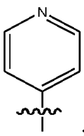, 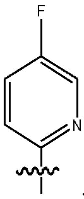, 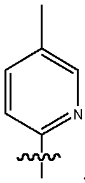, 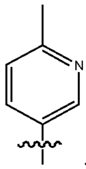, 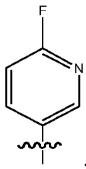, 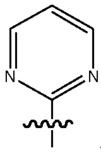,

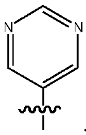, 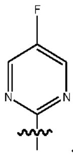, 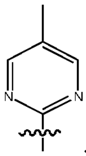, 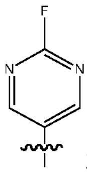;

when X is N, P is as defined above; or
when X is CH, P is as defined above.

Embodiment 6: the compound of formula I or a pharmaceutically acceptable salt thereof as described in Embodiment 1, characterized in that, in the compound of formula I, X and P are selected independently from each other, and:

X is selected from CH or N;
P is selected from substituted or unsubstituted phenyl, substituted or unsubstituted five-membered heteroaryl

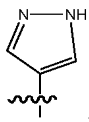,

substituted or unsubstituted six-membered heteroaryl

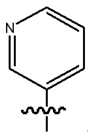,

for the above phenyl, the substituent is selected from 1 or 2 of: methyl, fluorine;
for the above five-membered heteroaryl, the substituent is methyl;
for the above six-membered heteroaryl, the substituent is methyl;
preferably, P is selected from:

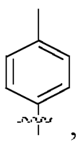, 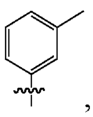, 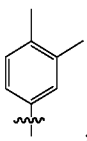, 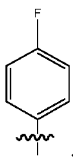, 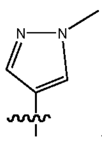, 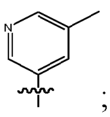;

when X is N, P is as defined above; or
when X is CH, P is as defined above.

Embodiment 7: the novel aromatic ring derivative containing an amide substitution or a pharmaceutically acceptable salt thereof as described in any of Embodiments 1-5, characterized in that, the compound of formula I is selected from compounds 1-106.

Embodiment 8: the novel aromatic ring derivative containing an amide substitution or a pharmaceutically acceptable salt thereof as described in Embodiment 7, characterized in that, the compound of formula I is selected from: compound 2, compound 3, compound 4, compound 5, compound 34, compound 38, compound 54, compound 55, compound 57, compound 62, compound 86, compound 91.

In a second aspect, Embodiment 9: a pharmaceutical composition, characterized in that, comprising the novel aromatic ring derivative containing an amide substitution or a pharmaceutically acceptable salt thereof as described in any of Embodiments 1-8 and optionally one or more pharmaceutically acceptable carriers, diluents, excipients, or adjuvants is provided.

In a third aspect, Embodiment 10: use of the novel aromatic ring derivative containing an amide substitution or a pharmaceutically acceptable salt thereof as described in any of Embodiments 1-8 or the pharmaceutical composition as described in Embodiment 9 in the manufacture of a medicament for the treatment of TYK 2-mediated related diseases is provided;

Preferably, the disease is selected from inflammatory or autoimmune diseases;

Further preferably, the disease is selected from psoriasis, psoriatic arthritis, dermatitis, lupus erythematosus, inflammatory bowel disease, hidradenitis suppurativa, rheumatoid arthritis or uveitis.

Embodiment 11: a method for the treatment of TYK 2-mediated related diseases, comprising administering to a subject in need the novel aromatic ring derivative containing an amide substitution or a pharmaceutically acceptable salt thereof as described in any of Embodiments 1-8 or the pharmaceutical composition as described in Embodiment 9;

Preferably, the disease is selected from inflammatory or autoimmune diseases;

Further preferably, the disease is selected from psoriasis, psoriatic arthritis, dermatitis, lupus erythematosus, inflammatory bowel disease, hidradenitis suppurativa, rheumatoid arthritis or uveitis.

Embodiment 12: the novel aromatic ring derivative containing an amide substitution or a pharmaceutically acceptable salt thereof as described in any of Embodiments 1-8 or the pharmaceutical composition as described in Embodiment 9, for the treatment of TYK 2-mediated related diseases;

Preferably, the disease is selected from inflammatory or autoimmune diseases;
Further preferably, the disease is selected from psoriasis, psoriatic arthritis, dermatitis, lupus erythematosus, inflammatory bowel disease, hidradenitis suppurativa, rheumatoid arthritis or uveitis.

In addition, the present invention further relates to the following embodiments:

Embodiment 1A: the present invention provides a novel aromatic ring derivative containing an amide substitution or a pharmaceutically acceptable salt thereof, wherein the compound is expressed by formula I:

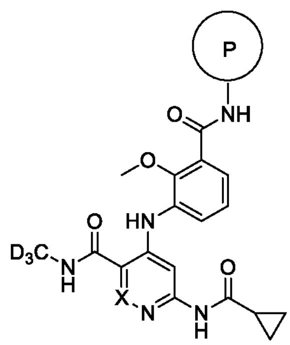

(I)

wherein, in the compound of formula I, X and P are selected independently from each other, and:

X is selected from CH, N;

P is selected from tert-butyl, substituted or unsubstituted phenyl, substituted or unsubstituted five-membered heteroaryl, substituted or unsubstituted six-membered heteroaryl, wherein the substituent is selected from C1-6 alkyl or C3-6 cycloalkyl, halogen, $-OCH_3$, $-CF_3$, -CN.

Embodiment 2A: in the compound of formula I as described in Embodiment 1A, X and P are selected independently from each other, and:

X is selected from CH, N;

P is selected from tert-butyl, substituted or unsubstituted phenyl, substituted or unsubstituted five-membered heteroaryl containing 2 to 3 N, substituted or unsubstituted six-membered heteroaryl containing 1 to 2 N, wherein the substituent is selected from C1-4 alkyl or C3-6 cycloalkyl, -F, $-OCH_3$, $-CF_3$, -CN.

Embodiment 3A: the compound of formula I as described in Embodiment 1A or 2A, which is selected from the following compounds 1-106:

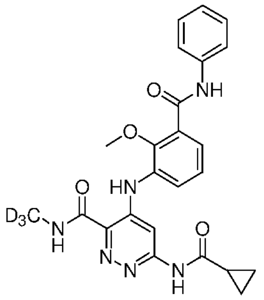

1

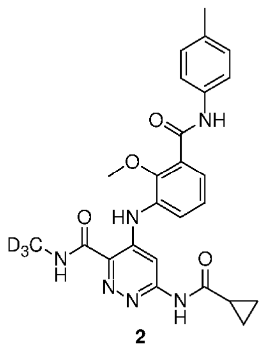

2

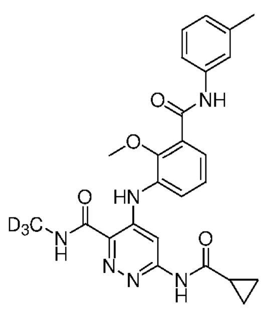

3

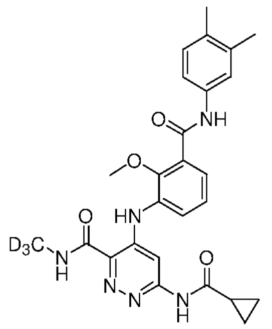

4

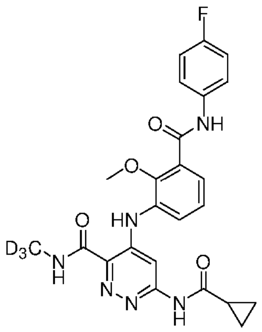

5

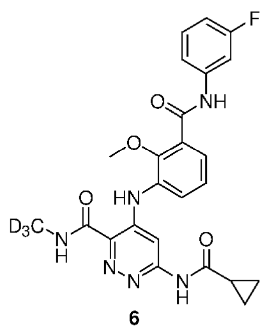

6

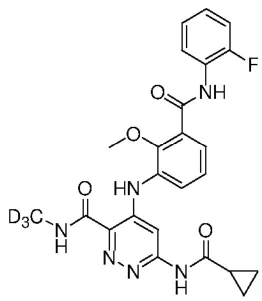

7

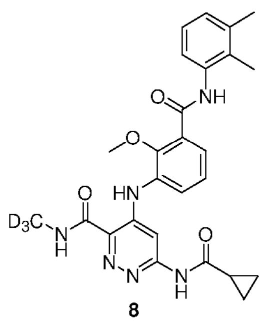

8

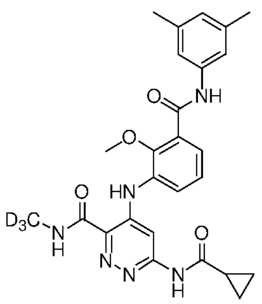

9

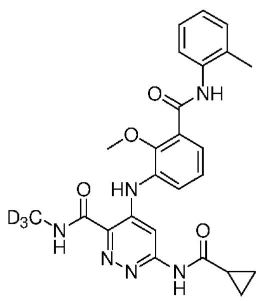

10

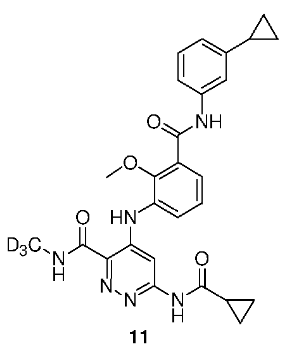

11

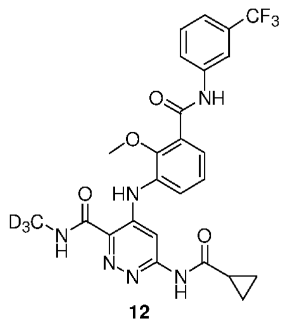

12

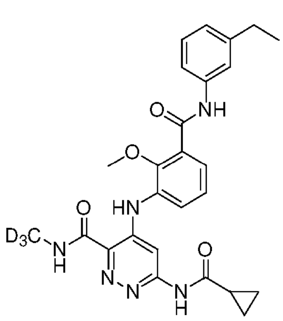

13

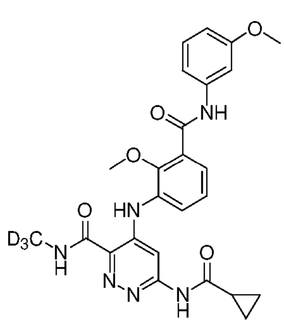

14

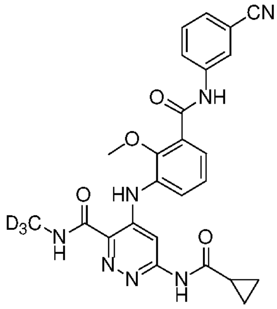

15

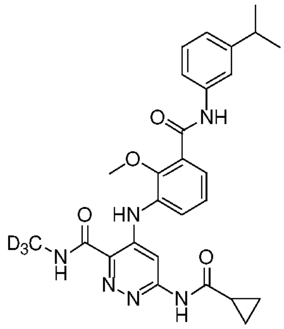

16

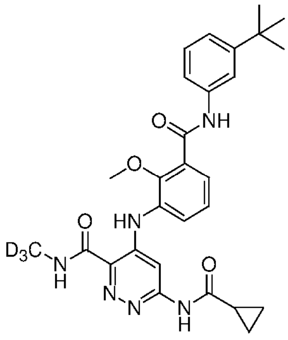

17

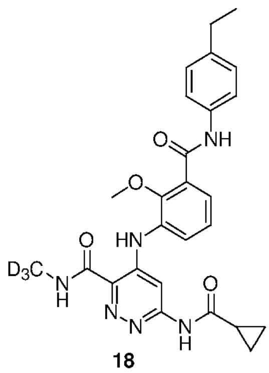

18

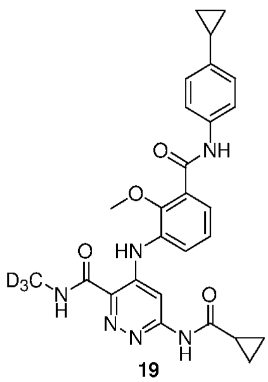

19

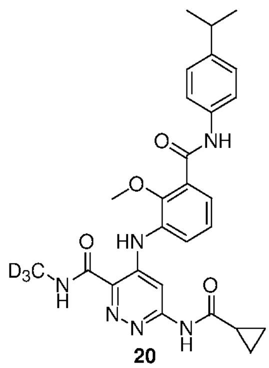

20

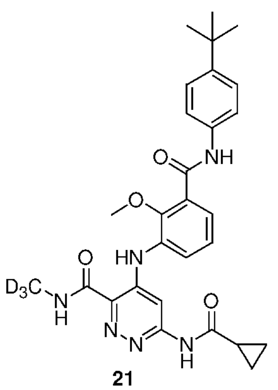

21

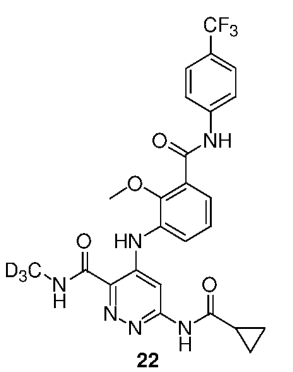

22

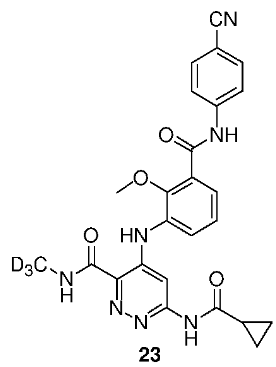

23

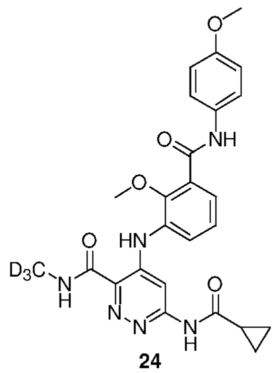

24

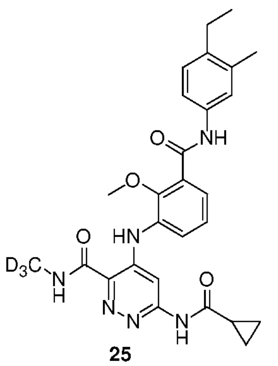

25

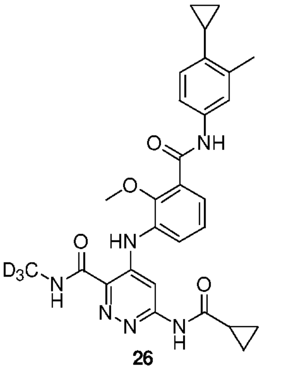

26

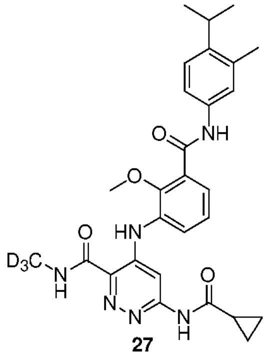

27

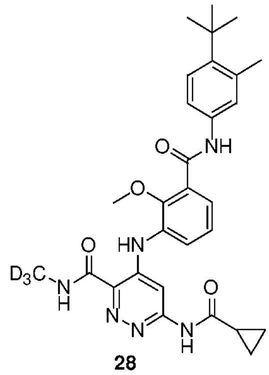

28

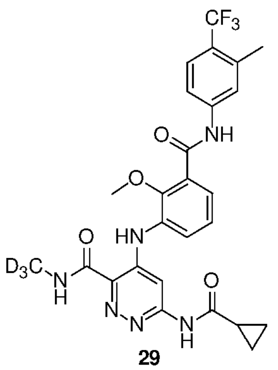

29

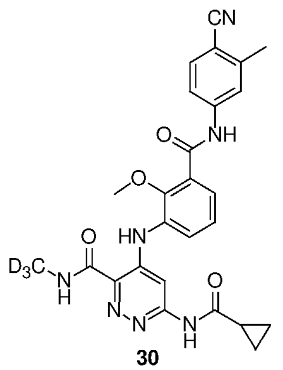

30

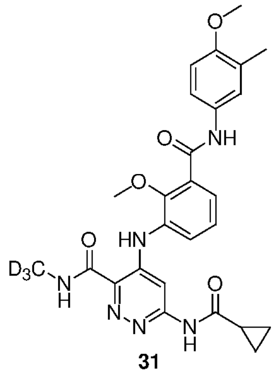

31

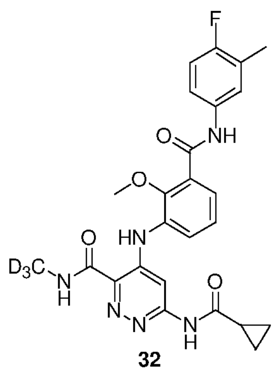

32

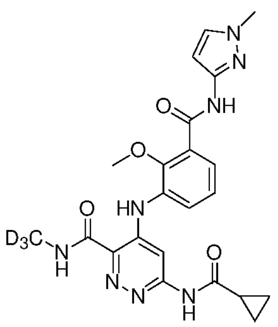

33

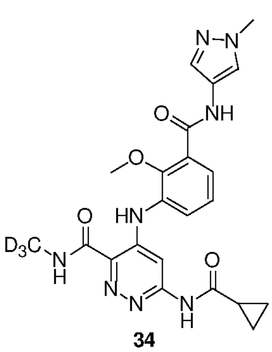

34

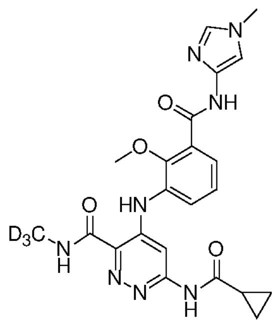

35

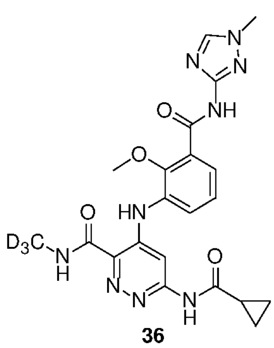

36

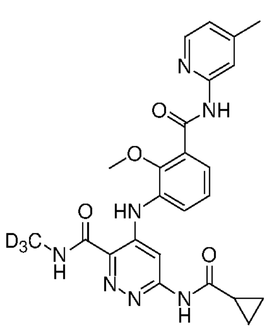

37

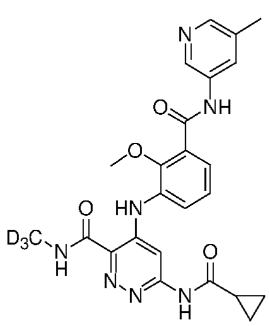

38

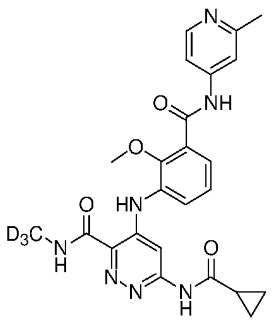

39

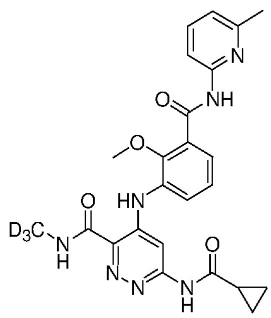

40

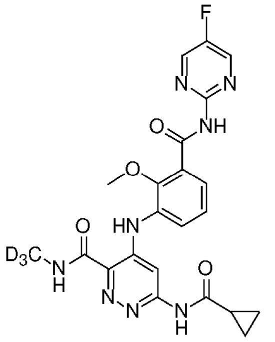

41

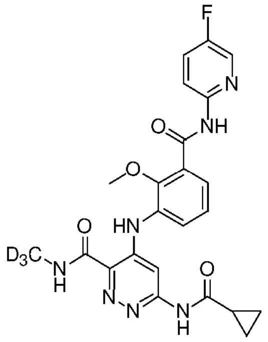

42

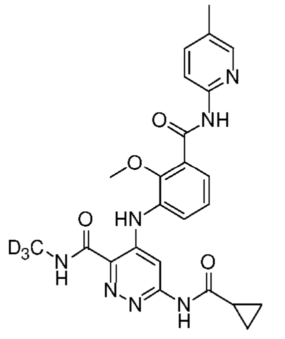

43

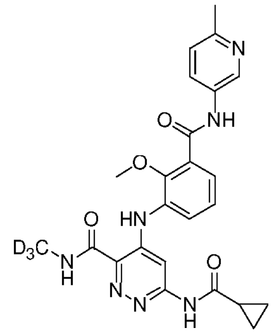

44

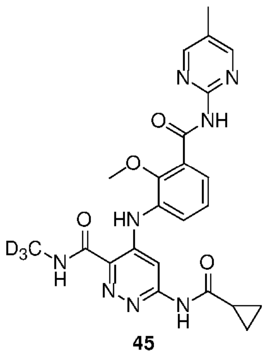

**45**

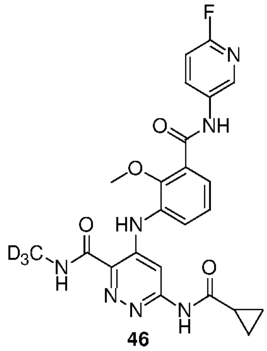

**46**

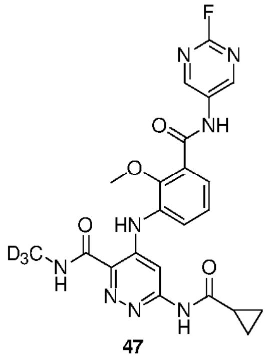

**47**

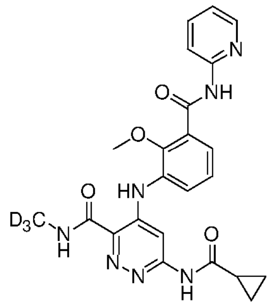

**48**

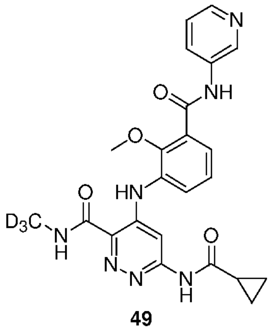

**49**

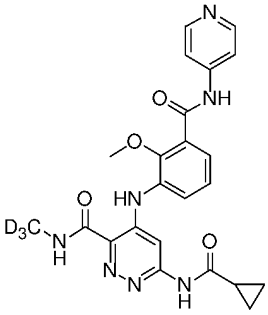

**50**

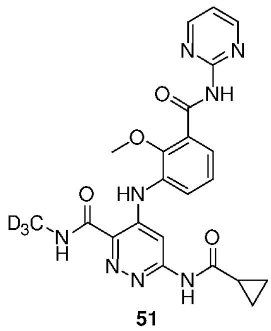

**51**

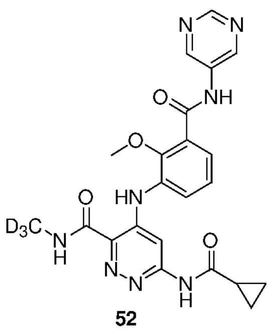

**52**

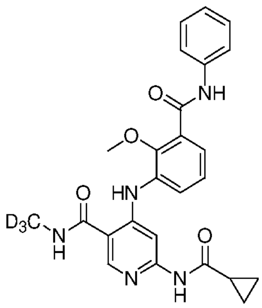

**53**

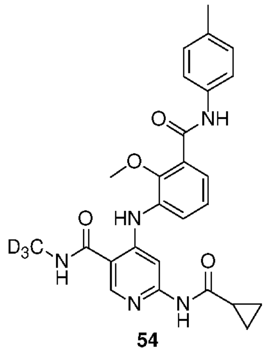

**54**

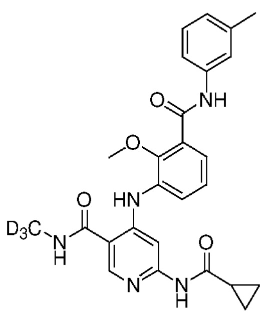

**55**

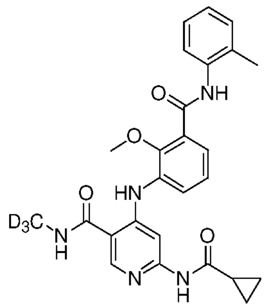

**56**

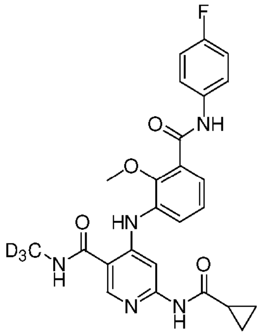

**57**

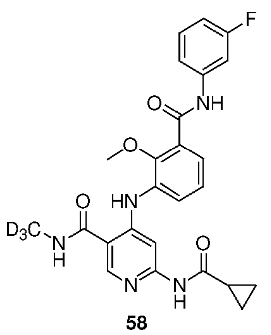

**58**

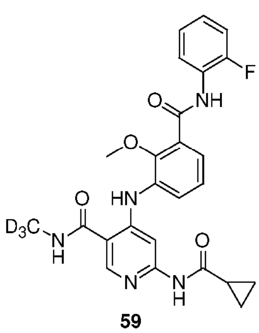

**59**

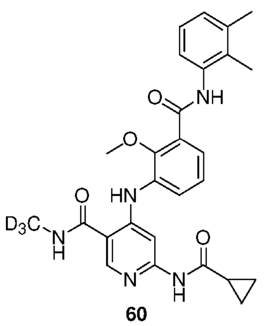

**60**

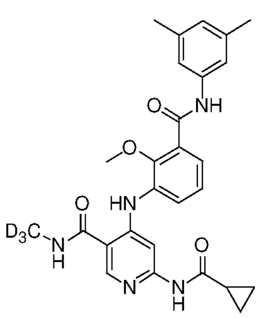

**61**

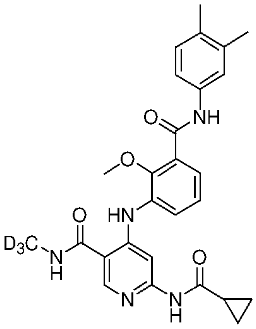

**62**

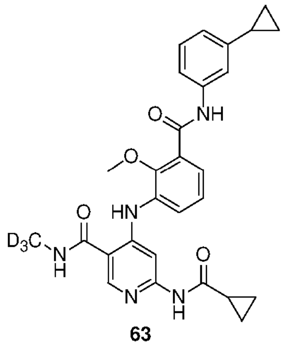

**63**

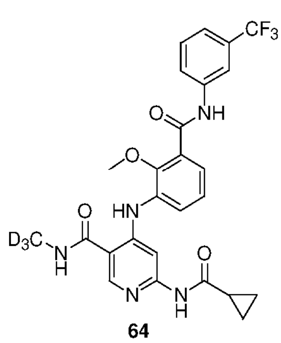

**64**

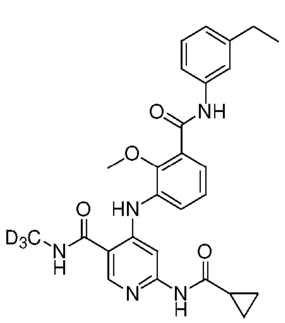

**65**

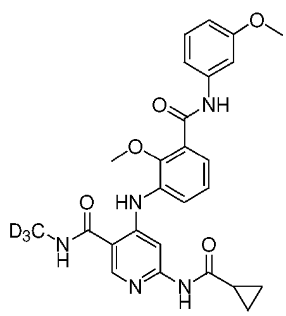

**66**

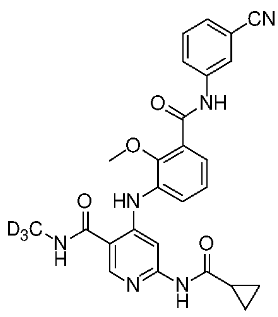

**67**

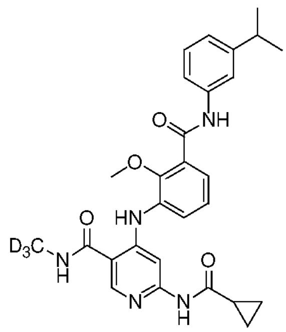

**68**

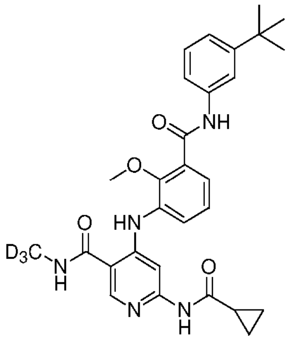

**69**

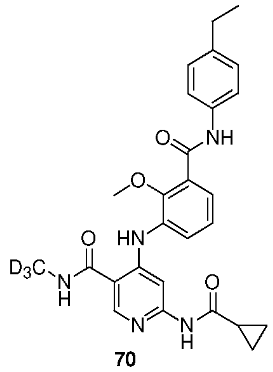

**70**

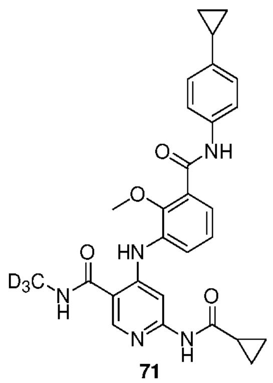

**71**

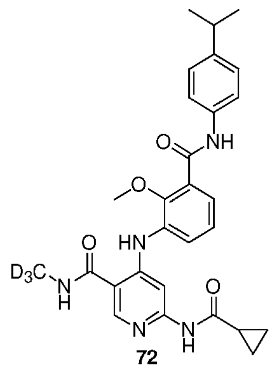

**72**

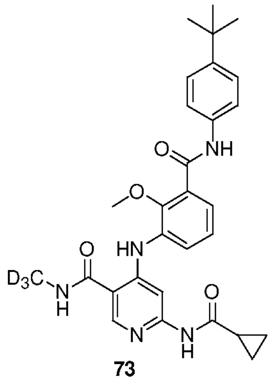

**73**

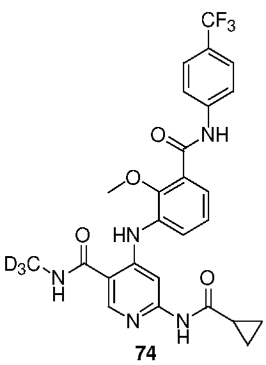

**74**

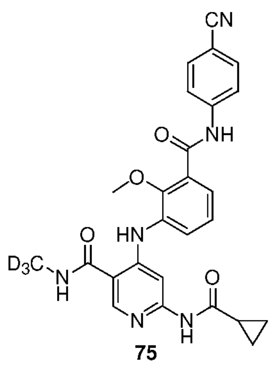

**75**

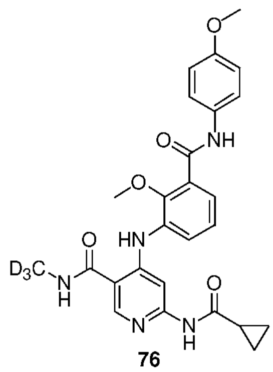

**76**

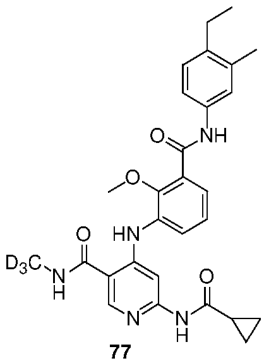
77

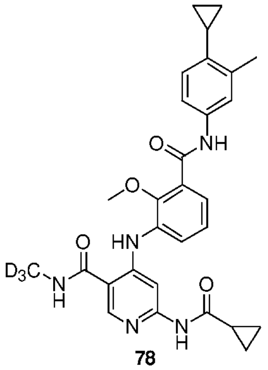
78

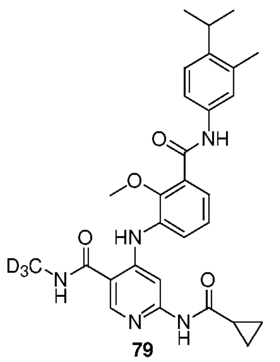
79

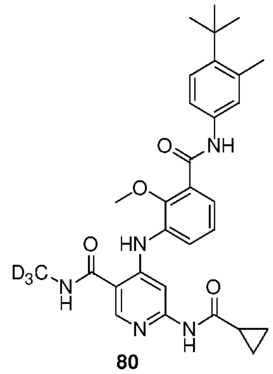
80

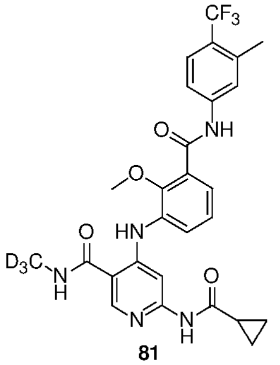
81

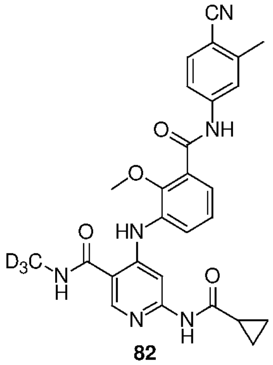
82

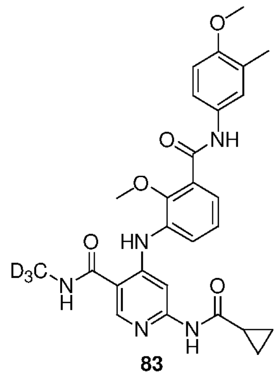
83

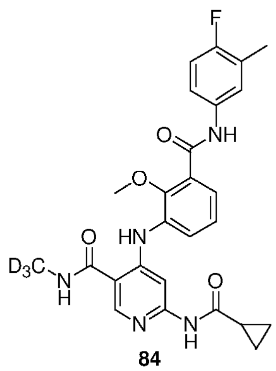
84

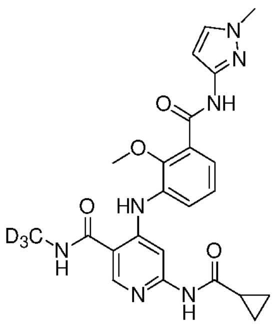
85

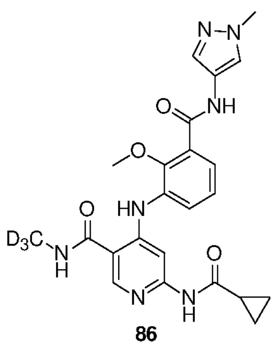
86

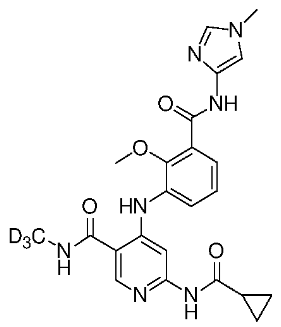
87

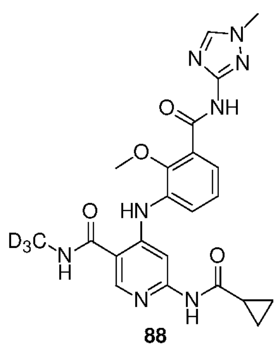
88

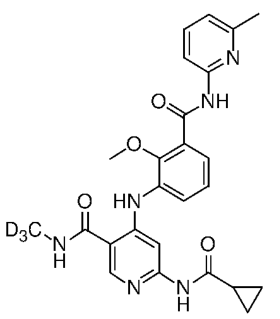
89

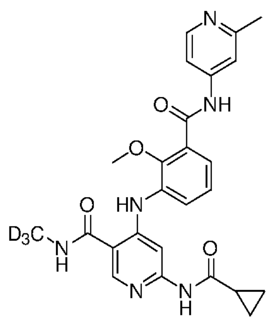
90

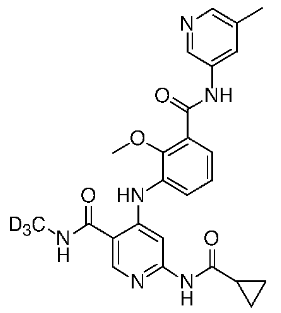
91

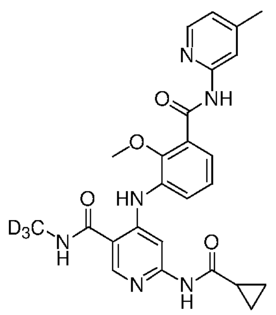
92

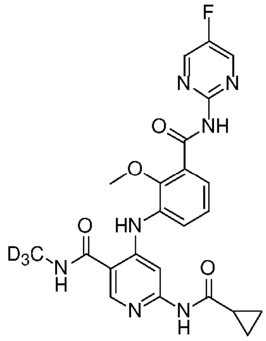

**93**

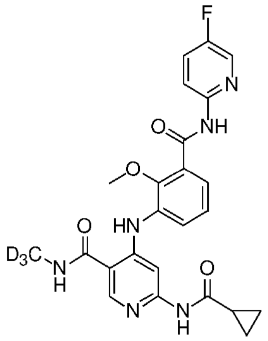

**94**

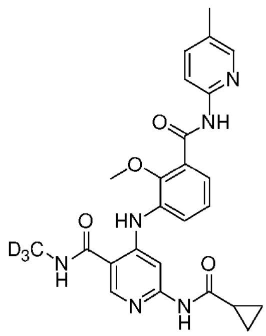

**95**

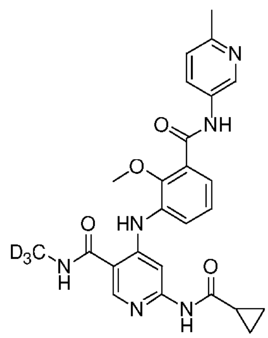

**96**

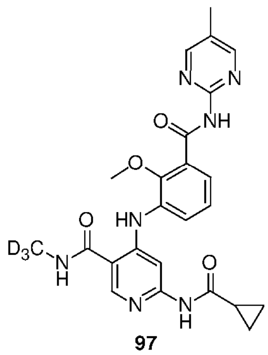

**97**

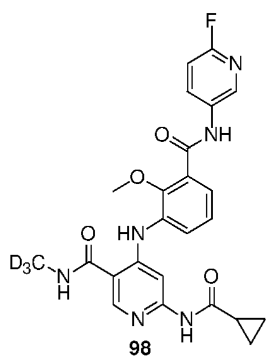

**98**

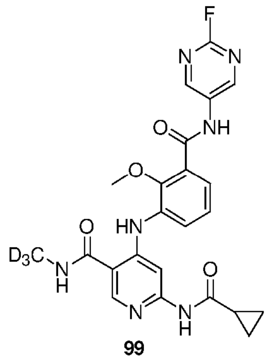

**99**

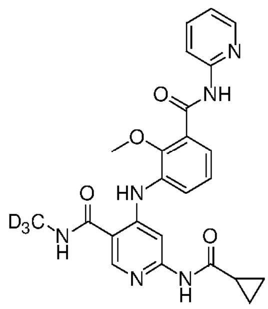

**100**

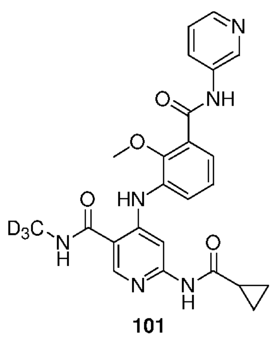

**101**

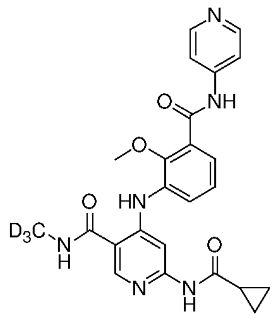

**102**

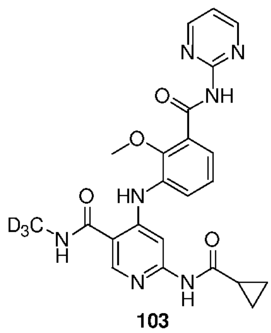

**103**

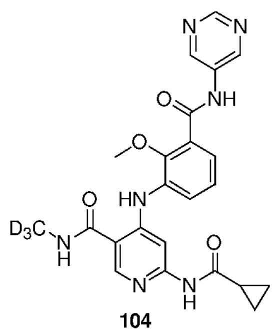

**104**

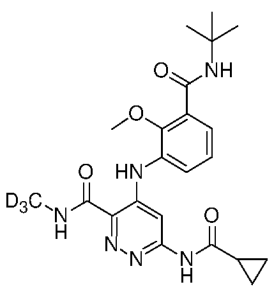

**105**

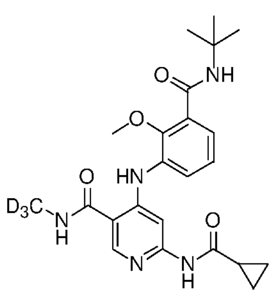

**106**

The compounds and intermediates of the present invention may also exist in different tautomeric forms, and all such forms are encompassed within the scope of the present invention. The term "tautomer" refers to the structural isomers of different energies that can be interconverted through low energy barriers.

The pharmaceutically acceptable salts of the compounds of the present invention refer to conventional non-toxic salts formed from pharmaceutically acceptable inorganic or organic acids.

The present invention also includes compounds of the present invention labeled with isotopes that are the same as those described herein, but with one or more atoms replaced by atoms with atomic weights or mass numbers different from those commonly found in nature. Examples of isotopes that can be incorporated into the compounds of the present

invention include, but are not limited to, isotopes of hydrogen ($^{2}H$, $^{3}H$), carbon ($^{11}C$, $^{13}C$, $^{14}C$), nitrogen ($^{13}N$, $^{15}N$), oxygen ($^{15}O$, $^{17}O$, $^{18}O$), phosphorus ($^{31}P$, $^{32}P$), sulfur ($^{35}S$), fluorine ($^{18}F$), iodine ($^{123}I$, $^{125}I$), and chlorine ($^{36}Cl$).

In addition, substitution with heavier isotopes such as deuterium (i.e., $^{2}H$) may afford certain therapeutic advantages resulting from higher metabolic stability (for example increased in vivo half-life or reduced dosage requirements) and may be preferred in certain circumstances, wherein deuterium substitution may be partial or complete, and partial deuterium substitution means substitution of at least one hydrogen with at least one deuterium, all such forms of the compound being encompassed within the scope of the present invention.

Embodiment 4A: the present invention further provides a pharmaceutical composition, comprising the novel aromatic ring derivative containing an amide substitution or a pharmaceutically acceptable salt thereof as described in any of Embodiments 1A-3A and optionally one or more pharmaceutically acceptable carriers, diluents, excipients or adjuvants.

Embodiment 5A: the present invention provides use of the novel aromatic ring derivative containing an amide substitution or a pharmaceutically acceptable salt thereof as described in any of Embodiments 1A-3A or the pharmaceutical composition comprising the novel aromatic ring derivative containing an amide substitution or a pharmaceutically acceptable salt thereof as described in Embodiment 4A in the manufacture of a medicament for the treatment of TYK 2-mediated related diseases.

Embodiment 6A: the use as described in Embodiment 5A, wherein the disease is selected from inflammatory or autoimmune diseases.

Embodiment 7A: the use as described in Embodiment 5A or 6A, wherein the disease includes, but is not limited to, psoriasis, psoriatic arthritis, dermatitis, lupus erythematosus, inflammatory bowel disease, hidradenitis suppurativa, rheumatoid arthritis or uveitis; preferably, psoriasis, psoriatic arthritis, atopic dermatitis, dermatomyositis, systemic lupus erythematosus, cutaneous lupus erythematosus, lupus nephritis, hidradenitis suppurativa, Crohn's disease, ulcerative colitis, rheumatoid arthritis or uveitis.

Compared with the prior art, the present invention has the following beneficial effects:

The aromatic ring derivative containing an amide substitution provided in the present invention, as a novel TYK2 inhibitor, has a good TYK2 inhibitory effect, has a good therapeutic effect in an in vivo model of psoriasis and asthma animals, and has good druggability, high stability and good safety.

**Detailed description of specific embodiments**

In the present invention, unless otherwise explicitly indicated, the description "... selected independently from each other " as used throughout this document may mean that the particular options expressed between the same or different symbols do not affect each other in different groups, or that the particular options expressed between the same or different symbols do not affect each other in the same group.

The substituents of the compound of the present invention are disclosed according to the type or range of the group. In particular, the present invention includes each independent secondary combination of each member of these group types and ranges. For example, the term "C1-C6 alkyl" specifically refers to the independently disclosed methyl, ethyl, C3 alkyl, C4 alkyl, C5 alkyl and C6 alkyl.

Unless otherwise indicated, the above groups and substituents have the ordinary meaning in the field of medicinal chemistry.

The term "C1-C6 alkyl" refers to any straight or branched chain group containing 1 to 6 carbon atoms, such as methyl, ethyl, n-propyl, isopropyl, n-butyl, isobutyl, tert-butyl, sec-butyl, n-pentyl, tert-pentyl, n-hexyl and the like. Similarly, "C1-C4 alkyl" refers to any straight or branched chain group containing 1 to 4 carbon atoms, such as methyl, ethyl, n-propyl, isopropyl, n-butyl, isobutyl, tert-butyl, sec-butyl and the like.

The terms "alkoxy" and "alkyloxy" refer to any of the above alkyl groups (e.g., C1-C6 alkyl, C1-C4 alkyl, and the like) attached to the remainder of the molecule through an oxygen atom -O-, and are exemplified by C1-C6 alkoxy, C1-C4 alkoxy, and specifically, methoxy, ethoxy, and the like.

The term "haloalkyl" refers to any of the above alkyl groups (e.g., C1-C6 alkyl, C1-C4 alkyl, and the like) substituted with one or more (e.g., 2 to 13 or 2 to 9) halogen atoms (e.g., fluorine, chlorine, bromine, iodine), such as trifluoromethyl, difluoromethyl, dichloromethyl, trichloromethyl, iodomethyl, bromoethyl, 1,2-dichloroethyl, and the like.

The term "C3-C6 cycloalkyl" refers to a saturated hydrocarbon monovalent ring containing 3 to 6 ring carbon atoms, and the cycloalkyl can be in the form of a single ring, fused ring, bridged ring, and the like. Examples of exemplary cycloalkyl include, but are not limited to, the following:

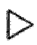 , 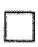 , 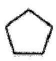 , 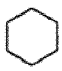 , 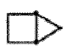 , 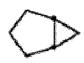 .

Examples of the term "C3-C6 cycloalkyl" include

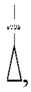, 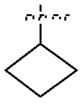, 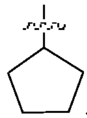, 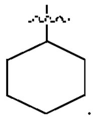.

Halogen refers to fluorine, chlorine, bromine or iodine.

The term "6- to 10-membered aryl" refers to an aromatic 6- to 10-membered monocyclic or bicyclic group. Examples include phenyl and naphthyl, preferably phenyl.

The term "five-/six-membered heteroaryl" refers to substituted and unsubstituted aromatic 5- or 6-membered monocyclic groups having at least one heteroatom (N, O, S or P) in at least one ring, the heteroatom-containing ring optionally further having 1, 2 or 3 heteroatoms selected from N, O, S or P.

Exemplary "five-membered/six-membered heteroaryl" groups include, but are not limited to, pyrrolyl/ring, pyrazolyl/ring, imidazolyl/ring, oxazolyl/ring, isoxazolyl/ring, thiazolyl/ring, thiadiazolyl/ring, isothiazolyl/ring, furyl/ring, thienyl/ring, oxadiazolyl/ring, pyridyl/ring, pyrazinyl/ring, pyrimidinyl/ring, pyridazinyl/ring, triazinyl/ring, triazolyl/ring, pyridazinyl/ring, 2-pyridone, and the like.

The term "substituted" means optionally substituted with 1 or more (e.g., 2 to 9, such as 2, 3, 4, 5, 6, 7, 8, 9) halogen atoms, C1-C6 alkyl, C3-C6 cycloalkyl, C1-C6 alkoxy, C1-C6 haloalkyl, cyano, and the like.

In the present invention, "treatment" generally means obtaining a desired pharmacological and/or physiological effect. The effect may be prophylactic in terms of completely or partially preventing the disease or symptoms thereof, and/or may be therapeutic in terms of partially or completely stabilizing or curing the disease and/or side effects due to the disease. As used herein, "treatment" encompasses any treatment of a disease in a patient, including: (a) preventing a disease or condition that occurs in a patient susceptible to the disease or condition but has not yet been diagnosed as having the disease; (b) inhibiting the symptoms of the disease, i.e., arresting its development; or (c) alleviating the symptoms of the disease, i.e., causing regression of the disease or symptoms.

In the present invention, "effective amount" means an amount effective to achieve a desired therapeutic or prophylactic effect at a necessary dose and time, the "therapeutically effective amount" of a substance/molecule of the present invention may vary depending on factors such as the disease state, age, gender, and body weight of the individual, and the ability of the substance/molecule to elicit a desired response in the individual. Therapeutically effective amount also encompasses an amount effective to achieve the therapeutically beneficial effect of the substance/molecule over any toxic or detrimental consequences. "Prophylactically effective amount" means an amount effective to achieve a desired prophylactic effect at a necessary dose and time. Typically, but not necessarily, a prophylactically effective amount will be lower than a therapeutically effective amount, since the prophylactic dose is administered to the subject prior to the onset of the disease or at an early stage of the disease. **In** the case of cancer, a therapeutically effective amount of a drug may reduce the number of cancer cells; reduce the tumor volume; inhibit (i.e., slow to some extent, preferably stop) cancer cell infiltration into peripheral organs; inhibit (i.e., slow to some extent, preferably stop) tumor metastasis; inhibit tumor growth to some extent; and/or to some extent alleviate one or more symptoms associated with cancer.

**In** the present invention, "subject" refers to a vertebrate. **In** some embodiments, the vertebrate is a mammal. Mammals include, but are not limited to, livestock (such as cattle), pets (such as cats, dogs, and horses), primates, mice, and rats. **In** some embodiments, the mammal refers to a human.

The pharmaceutical composition of the present invention can be prepared into various forms according to different administration routes. For example, the pharmaceutical composition may be administered in any of the following ways: oral, aerosol inhalation, rectal, nasal, vaginal, topical, parenteral such as subcutaneous, intravenous, intramuscular, intraperitoneal, intrathecal, intraventricular, intrasternal and intracranial injection or infusion, or via an explanted reservoir. Among them, the topical administration is preferred.

The compounds described in the present invention can optionally be used in combination with one or more other active ingredients, and their respective amounts and ratios can be adjusted by those skilled in the art according to specific diseases, patient conditions, and clinical needs.

As used herein, the term "pharmaceutically acceptable salt" refers to (i) salts formed by acidic functional groups present in the compounds provided by the present invention and appropriate inorganic or organic cations (bases), including but not limited to alkali metal salts, such as sodium salts, potassium salts, lithium salts and the like; alkaline earth metal salts, such as calcium salts, magnesium salts and the like; other metal salts, such as aluminum salts, iron salts, zinc salts, copper salts, nickel salts, cobalt salts and the like; inorganic base salts, such as ammonium salts; organic base salts, such as tert-octyl amine salts, dibenzylamine salts, morpholine salts, glucosamine salts, phenylglycinatealkyl ester salts, ethylenediamine salts, N-methylglucosamine salts, guanidine salts, diethylamine salts, triethylamine salts, dicyclohexylamine salts, N,N'-dibenzylethylenediamine salts, diethanolamine salts, N-benzyl-phenethylamine salts, piperazine salts, tetramethylamine salts, and tris(hydroxymethyl) aminomethane salts. And, (ii) salts formed by alkaline functional

groups present in the compounds provided by the present invention and appropriate inorganic or organic anions (acids), including but not limited to hydrohalic acid salts, such as hydrofluoric acid salts, hydrochloric acid salts, hydrobromic acid salts, hydroiodic acid salts and the like; inorganic acid salts, such as nitrates, perchlorates, sulfates, phosphates and the like; lower alkyl sulfonates, such as methanesulfonates, trifluoromethanesulfonates, ethanesulfonates and the like; arylsulfonates, such as benzenesulfonates, para-benzenesulfonates and the like; organic acid salts, such as acetates, malates, fumarates, succinates, citrates, tartrates, oxalates, maleates and the like; amino acid salts, such as glycinates, trimethylglycinates, arginates, ornithinates, glutamates, aspartates and the like.

The embodiments of the present invention will be described in detail with reference to examples, but it will be understood by those skilled in the art that the following examples are only illustrative of the present invention and should not be construed as limiting the scope of the present invention. Where the specific conditions are not specified, the examples are carried out under conventional conditions. The reagents or instruments used, whose manufacturers are not specified, are all conventional products that are commercially available.

The structures of the compounds of the present invention were determined by liquid chromatography-mass spectrometry (LC-MS) and nuclear magnetic resonance (NMR). The determination of LC-MS was carried out using an AB Sciex TripleTOF 4600 mass spectrometer, and a Shimadzu LC-20AD XR high-performance liquid chromatograph, with Agela Venusil MP C18 (2.1x50, 3um) as the chromatographic column. The determination of nuclear magnetic resonance was carried out using a BRUKER AVANCE NEO 400MHz nuclear magnetic resonance spectrometer.

Unless otherwise specified, all reactions of the present invention were carried out under continuous magnetic stirring in dry nitrogen or argon. The solvent was a pre-dried solvent and the reaction temperature is Celsius.

**Example** 1

6-(Cyclopropaneamido)-4-(2-methoxy-3-(benzoylanilino)-N-(methyl-d3)pyridazine-3-carboxamide

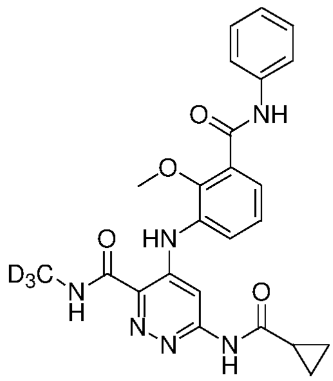

**1**

Step 1

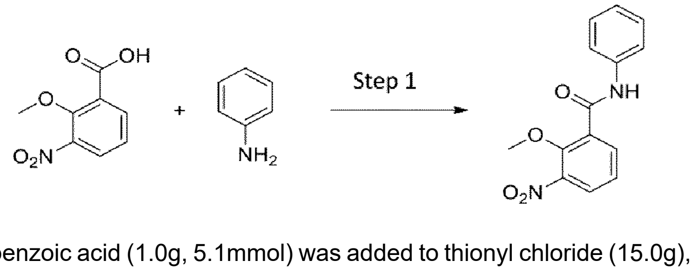


2-Methoxy-3-nitrobenzoic acid (1.0g, 5.1mmol) was added to thionyl chloride (15.0g), and reacted under reflux with stirring and heating until completion of the reaction as detected by TLC. The reaction solution was cooled to room temperature and concentrated under reduced pressure. The residue was dissolved with 5mL of acetone, and added dropwise to a solution of aniline (0.4g, 4.6mmol) and triethylamine (1.0g, 10.2mmol) in 10mL of acetone, while controlling the temperature to be less than 10°C. After completion of dropping, the resulting solution was allowed to react at room temperature until the reaction was completed as detected by TLC, to which 50mL of water was added, followed by filtering,

drying at room temperature for 24h, to obtain 2-methoxy-3-nitro-N-benzoylaniline (1.1g, 71.9%).

MS m/z (ESI): 273.08[M+H]$^+$.

**Step 2**

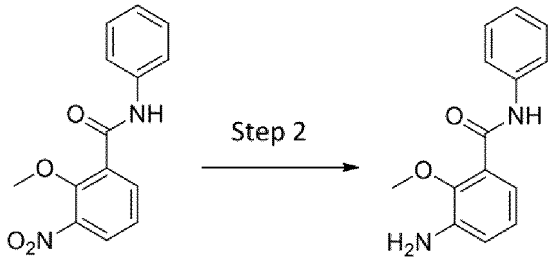


2-Methoxy-3-nitro-N-benzoylaniline (1.0g, 3.7mmol) and 10% Pd/C (0.15g) were added to 30mL of methanol, hydrogen was charged, and the resulting solution was allowed to react at 25°C under a hydrogen atmosphere until the reaction was completed as detected by TLC. The reaction solution was filtered, and the filtrate was evaporated to dryness to obtain 3-amino-2-methoxy-N-benzoylaniline (0.81g, 91.0%).

MS m/z (ESI): 243.11[M+H]$^+$.

Step 3

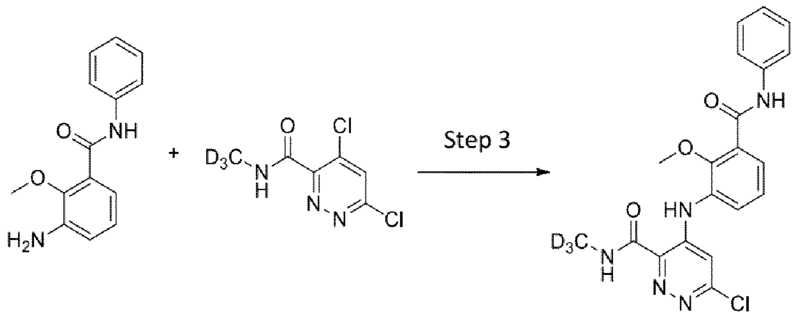


At room temperature, to a solution of 3-amino-2-methoxy-N-benzoylaniline (0.8g, 3.3mmol) and 4,6-dichloro-N-(methyl-d3)pyridazine-3-carboxamide (0.68g, 3.3mmol) in tetrahydrofuran (THF) (5mL), a solution of bis(trimethylsilyl) amino lithium (1M, 9.9mL, 9.9mmol) in THF was added dropwise. The resulting solution was allowed to react at room temperature until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-chloro-4-((2-methoxy-3-(benzoylanilino)-N-(methyl-d3)pyridazine-3-carboxamide (0.89g, 55%).

MS m/z (ESI): 415.81[M+H]$^+$.

**Step 4**

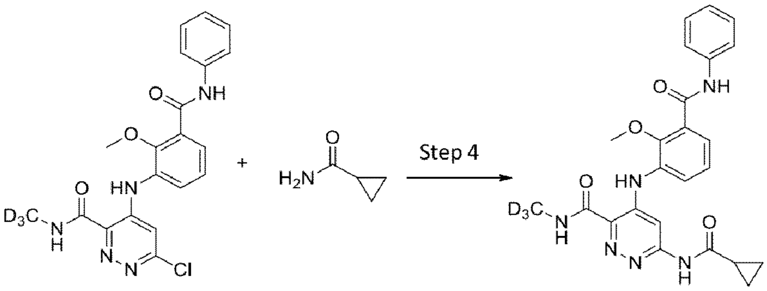


6-Chloro-4-((2-methoxy-3-(benzoylanilino)-N-(methyl-d3)pyridazine-3-carboxamide (0.8g, 1.9mmol), cyclopropanecarboxamide (0.16g, 1.9mmol), tri(dibenzylideneacetone) dipalladium (0.1g, 0.1mmol), 4,5-diphenylphosphine-9,9-dimethyloxaanthracene (0.12g, 0.2mmol) and cesium carbonate (1.27g, 3.9mmol) were added to 1,4-dioxane (10mL), and reacted under reflux with heating until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-(cyclopropaneamido)-4-(2-methoxy-3-(benzoylanilino)-N-(methyl-d3)pyridazine-3-carboxamide (0.43g, 48%).

MS m/z (ESI): 464.20$[M+H]^+$.

$^1$H-NMR(DMSO-$d_6$)δ:11.34(1H,s),10.34(1H,s),9.14(1H,s),8.16(1H,s),7.73-7.71(2H,m),7.58-7.56(1H, m),7.35-7.26(5H,m),7.11-7.07(1H,m),3.74(3H,s),2.08-2.06(1H,m),0.83-0.81(4H,m).

**Example 2**

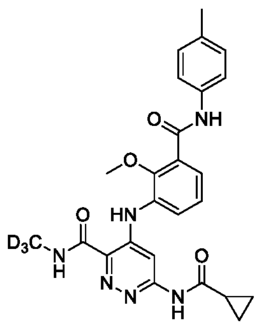

2

With reference to the synthetic method of Example 1, 4-methylaniline was used in place of aniline in Step 1, to obtain compound 2.

MS m/z (ESI): 478.22$[M+H]^+$.

$^1$H-NMR(DMSO-$d_6$)δ:11.34(1H,s),10.97(1H,s), 10.25(1H,s), 9.14(1H,s), 8.16(1H,s), 7.55-7.62(3H,m), 7.25-7.34 (2H,m), 7.13-7.15(2H, m), 3.76(3H,s), 2.26(3H,m), 2.07-2.09(1H,m), 0.81-0.83(4H,m).

**Example 3**

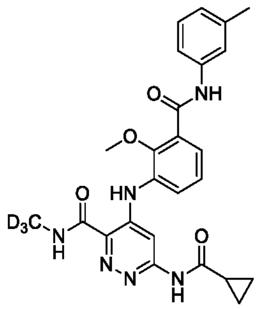

3

With reference to the synthetic method of Example 1, 3-methylaniline was used in place of aniline in Step 1, to obtain compound 3.

MS m/z (ESI): 478.22[M+H]+.

$^1$H-NMR(DMSO-$d_6$)δ:11.34(1H,s),10.97(1H,s), 10.25(1H,s), 9.14(1H,s), 8.16(1H,s), 7.55-7.62(3H,m), 7.25-7.34 (2H,m), 7.13-7.15(2H,m), 3.74(3H,s), 2.26(3H,m), 2.07-2.09(1H,m), 0.81-0.83(4H,m).

**Example 4**

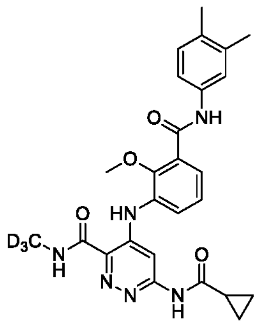

4

With reference to the synthetic method of Example 1, 3,4-dimethylaniline was used in place of aniline in Step 1, to obtain compound 4.

MS m/z (ESI): 492.21[M+H]+.

$^1$H-NMR(DMSO-$d_6$)δ:11.34(1H,s),10.97(1H,s), 10.17(1H,s), 9.14(1H,s), 8.16(1H,s), 7.55-7.57(1H,m), 7.50-7.51 (1H,m), 7.41-7.44(1H, m), 7.25-7.34(2H,m), 7.07-7.09(1H,m), 3.75(3H,s), 2.17-2.20(6H,m), 2.06-2.09(1H,m), 0.81-0.83(4H,m).

**Example 5**

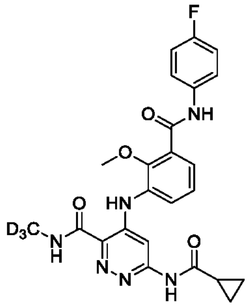

5

With reference to the synthetic method of Example 1, 4-fluoroaniline was used in place of aniline in Step 1, to obtain compound 5.

MS m/z (ESI): 482.20[M+H]$^+$.

$^1$H-NMR(DMSO-$d_6$)δ:11.34(1H,s),10.98(1H,s),10.41(1H,s),9.14(1H,s),8.16(1H,s),7.77-7.73(2H,m),7.65-7.56 (1H,m),7.53-7.04(4H,m),3.74(3H,s),2.08-2.06(1H,m),0.83(4H,d).

## Examples 6-32

With reference to the synthetic method of Example 1, aniline in Step 1 was correspondingly replaced to obtain compounds with structures listed in the following table:

| Example No. | Structural formula | m/z [M+H]$^+$ | Example No. | Structural formula | m/z [M+H]$^+$ |
|---|---|---|---|---|---|
| 6 | 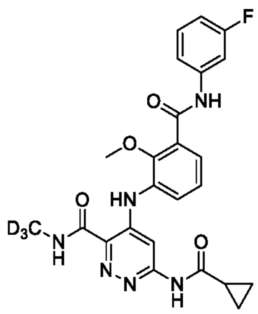 | 482.20 | 7 | 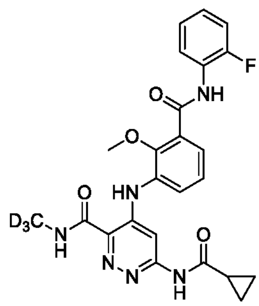 | 482.20 |
| 8 | 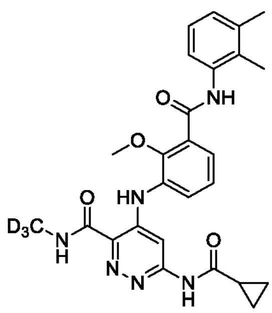 | 492.21 | 9 | 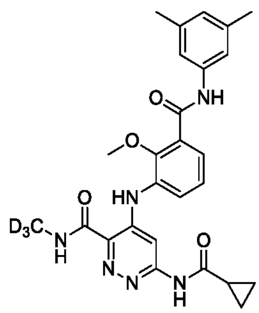 | 492.21 |

(continued)

| Example No. | Structural formula | m/z [M+H]+ | Example No. | Structural formula | m/z [M+H]+ |
|---|---|---|---|---|---|
| 10 | 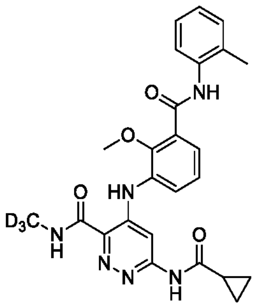 | 478.22 | 11 | 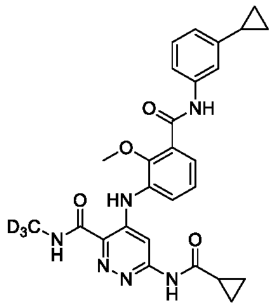 | 504.24 |
| 12 | 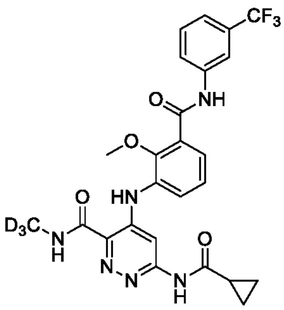 | 532.20 | 13 | 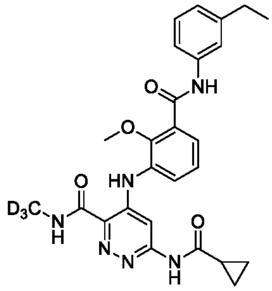 | 492.24 |
| 14 | 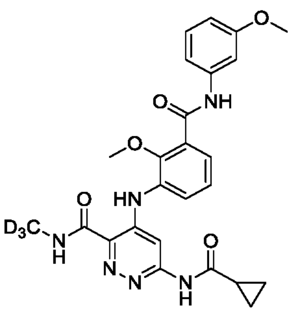 | 494.22 | 15 | 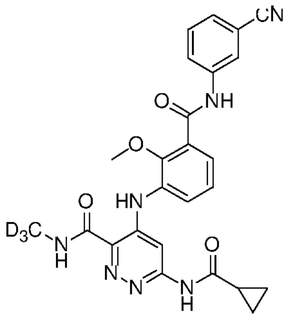 | 489.20 |
| 16 | 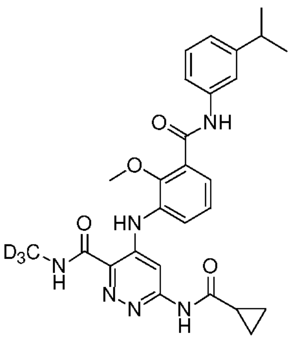 | 506.25 | 17 | 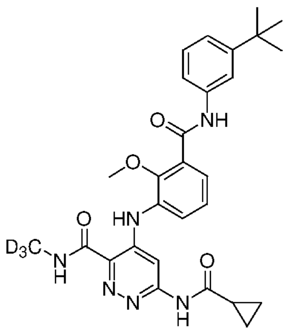 | 520.27 |
| 18 | 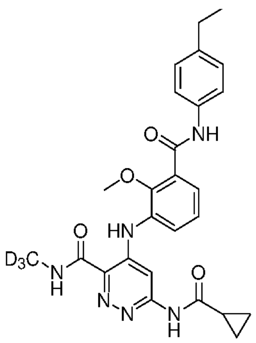 | 492.24 | 19 | 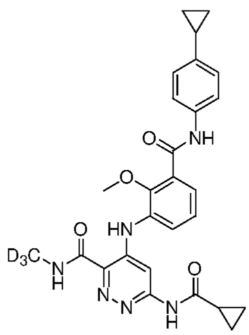 | 504.24 |

(continued)

| Example No. | Structural formula | m/z [M+H]⁺ | Example No. | Structural formula | m/z [M+H]⁺ |
|---|---|---|---|---|---|
| 20 | 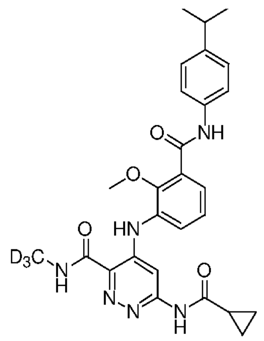 | 506.25 | 21 | 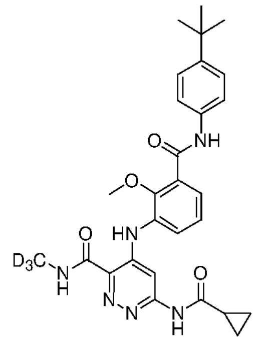 | 520.27 |
| 22 | 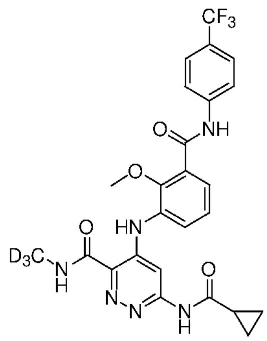 | 532.19 | 23 | 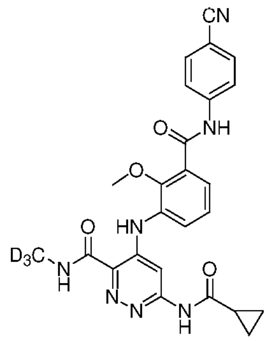 | 489.20 |
| 24 | 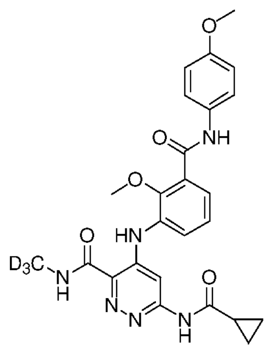 | 494.21 | 25 | 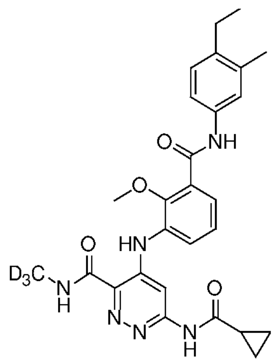 | 506.25 |
| 26 | 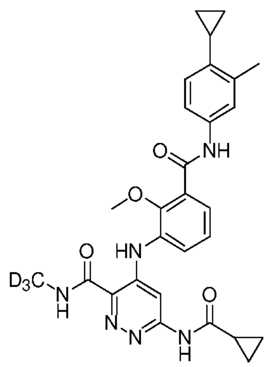 | 518.25 | 27 | 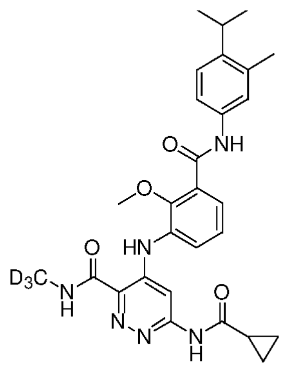 | 520.27 |

(continued)

| Example No. | Structural formula | m/z [M+H]+ | Example No. | Structural formula | m/z [M+H]+ |
|---|---|---|---|---|---|
| 28 | 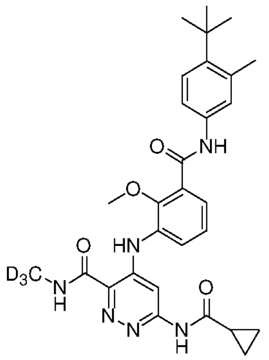 | 534.28 | 29 | 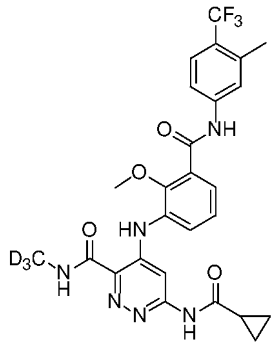 | 546.21 |
| 30 | 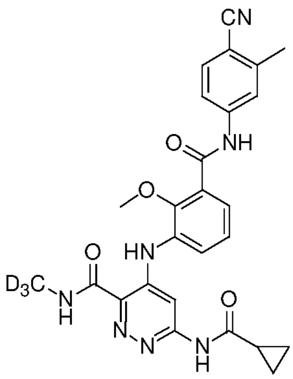 | 503.22 | 31 | 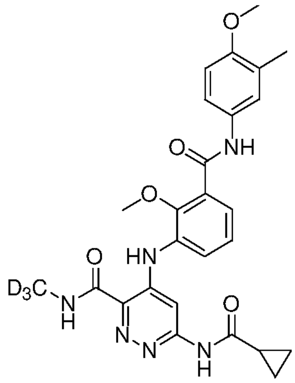 | 508.23 |
| 32 | 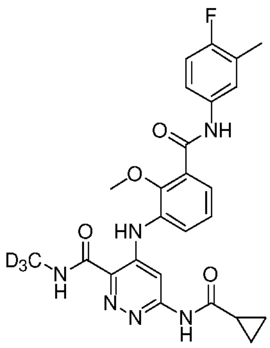 | 496.21 | | | |

**Example 33**

6-(Cyclopropanecarboxamido)-4-(2-methoxy-3-(1-methyl-1H-pyrazol-3-yl)carbamoyl)phenyl)amino)-N-(methyl-d3)pyridazine-3-carboxamide.

**Step 1**

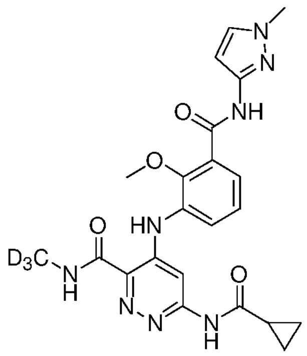

**33**

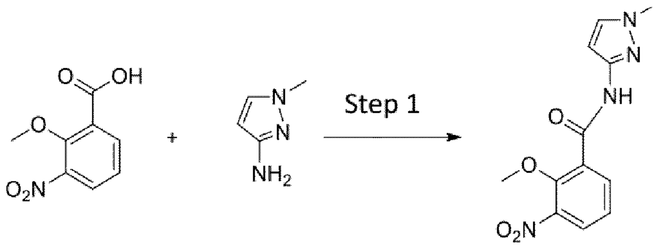


2-Methoxy-3-nitrobenzoic acid (1.0 g, 5.1mmol) was added to thionyl chloride (15.0g), and reacted under reflux with stirring and heating until the reaction was completed as detected by TLC. The reaction solution was cooled to room temperature and concentrated under reduced pressure. The residue was dissolved with 5mL of acetone, and added dropwise to a solution of 1-methyl-1H-pyrazole-3-amine (0.4g, 4.1mmol) and triethylamine (1.0g, 10.2mmol) in 10mL of acetone, while controlling the temperature to be less than 10°C. After completion of dropping, the resulting solution was allowed to react at room temperature until the reaction was completed as detected by TLC, to which 50mL of water was added, followed by filtering, drying at room temperature for 24h, to obtain 2-methoxy-N-(1-methyl-1H-pyrazol-3-yl)-3-nitrobenzamide (1.0g, 71.4%).

MS m/z (ESI): 277.09$[M+H]^+$.

**Step 2**

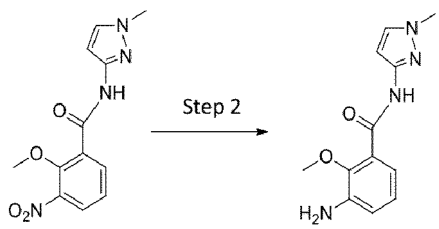


2-Methoxy-N-(1-methyl-1H-pyrazol-3-yl)-3-nitrobenzamide (0.9g, 3.2mmol) and 10% Pd/C (0.15g) were added to 30mL of methanol, and the resulting solution was allowed to react at 25°C under a hydrogen atmosphere until the reaction was completed as detected by TLC. The reaction solution was filtered, and the filtrate was evaporated to dryness to obtain 3-amino-2-methoxy-N-(1-methyl-1H-pyrazol-3-yl)benzamide (0.71g, 88.8%).

MS m/z (ESI): 247.10$[M+H]^+$.

**Step 3**

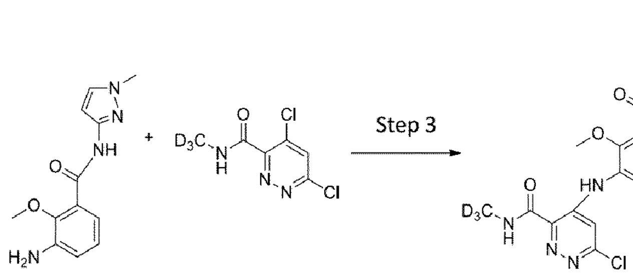


At room temperature, to a solution of 3-amino-2-methoxy-N-(1-methyl-1H-pyrazol-3-yl)benzamide (0.7g, 2.8mmol) and 4,6-dichloro-N-(methyl-d3)pyridazine-3-carboxamide (0.58g, 2.8mmol) in THF (5mL), a solution of bis(trimethylsilyl)amino lithium (1M, 8.4mL, 8.4mmol) in THF was added dropwise. The resulting solution was allowed to react at room temperature until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-chloro-4-((2-methoxy-3-((1-methyl-1H-pyrazol-3-yl)carbamoyl)phenyl)amino)-N-(methyl-d3)pyridazine (0.59g, 49.6%).

MS m/z (ESI): 419.14[M+H]+.

**Step 4**

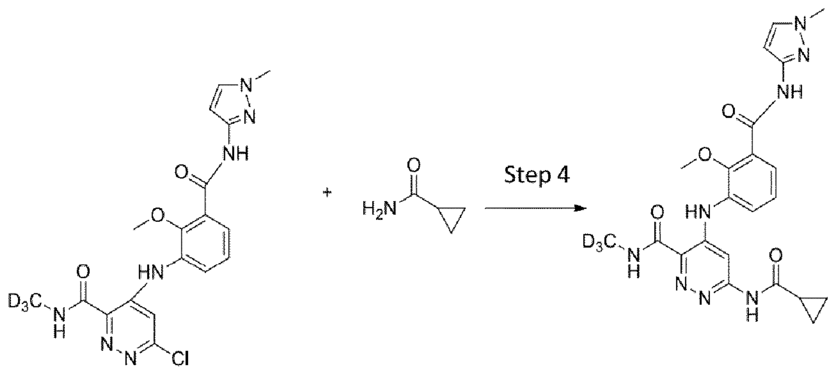


6-Chloro-4-((2-methoxy-3-((1-methyl-1H-pyrazol-3-yl)carbamoyl)phenyl)amino)-N-(methyl-d3)pyridazine-3-carboxamide (0.5g, 1.2mmol), cyclopropanecarboxamide (0.10g, 1.2mmol), tri(dibenzylideneacetone) dipalladium (0.1g, 0.1mmol), 4,5-diphenylphosphine-9,9-dimethyloxaanthracene (0.12g, 0.2mmol) and cesium carbonate (1.27g, 3.9mmol) were added to 1,4-dioxane (10mL), and reacted under reflux with heating until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-(cyclopropanecarboxamido)-4-(2-methoxy-3-(1-methyl-1H-pyrazol-3-yl)carbamoyl)phenyl)amino)-N-(methyl-d3)pyridazine-3-carboxamide (0.33g, 59.1%).

MS m/z (ESI): 468.22[M+H]+.

$^1$H-NMR(DMSO-$d_6$)δ:11.3(1H,s),10.93(1H,s),10.57(1H,s),9.14(1H,s),8.13(1H,s),7.60-7.54(2H, m),7.38-7.35(1H,m),7.28-7.24(1H,m),6.57-6.56(1H,m),3,75-3.72(6H,m),2.07-2.05(1H,m),0.82-0.80(4H,m).

**Example 34**

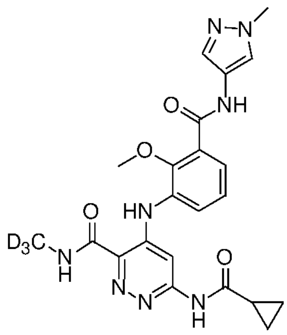

**34**

With reference to the synthetic method of Example 33, 1-methyl-1H-pyrazole-4-amine was used in place of 1-methyl-1H-pyrazole-3-amine in Step 1, to obtain compound 34.

MS m/z (ESI): 468.22[M+H]+.

$^1$H-NMR(DMSO-$d_6$)$\delta$:11.34(1H,s), 10.98(1H,s), 10.35(1H,s), 9.14(1H,s), 8.15(1H,s), 8.00(1H,s), 7.55-7.57(1H,m), 7.50 (1H,s), 7.31-7.33(1H,m), 7.24-7.28(1H,m), 3.80(3H,s), 3.71(3H,s), 2.06-2.08(1H,m), 0.80-0.83(4H,m).

**Examples 35-36**

With reference to the synthetic method of Example 33, 1-methyl-1H-pyrazole-3-amine in Step 1 was correspondingly replaced to obtain compounds with structures listed in the following table:

| Exam ple No. | Structural formula | m/z [M+H]+ | Examp le No. | Structural formula | m/z [M+H]+ |
|---|---|---|---|---|---|
| 35 | 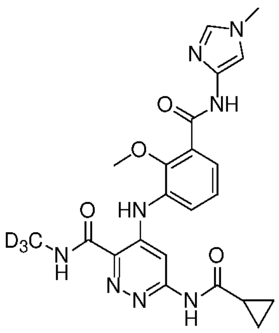 | 468.22 | 36 | 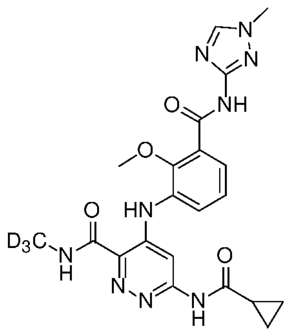 | 469.21 |

**Example 37**

6-(Cyclopropanecarboxamido)-4-(2-methoxy-3-(4-methylpyrid-2-yl)carbamoyl)phenyl)amino)-N-(methyl-d3)pyridazine-3-carboxamide.

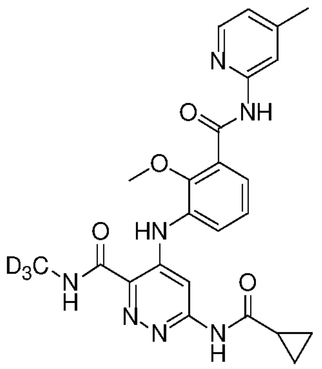

**37**

**Step 1**

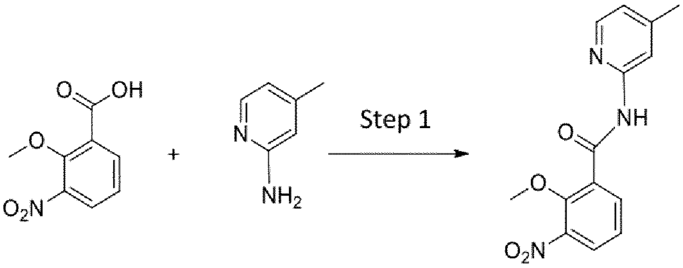


2-Methoxy-3-nitrobenzoic acid (1.0g, 5.1mmol) was added to thionyl chloride (15.0g), and reacted under reflux with stirring and heating until the reaction was completed as detected by TLC. The reaction solution was cooled to room temperature and concentrated under reduced pressure. The residue was dissolved with 5mL of acetone, and added dropwise to a solution of 4-methylpyridine-2-amine (0.5g, 4.6mmol) and triethylamine (1.0g, 10.2mmol) in 10mL of acetone, while controlling the temperature to be less than 10°C. After completion of dropping, the resulting solution was allowed to react at room temperature until the reaction was completed, as detected by TLC, to which 50mL of water was added, followed by filtering, drying at room temperature for 24h, to obtain 2-methoxy-N-(4-methylpyrid-2-yl)-3-nitrobenzamide (1.2g, 82.3%).

MS m/z (ESI): 288.12[M+H]$^+$.

**Step 2**

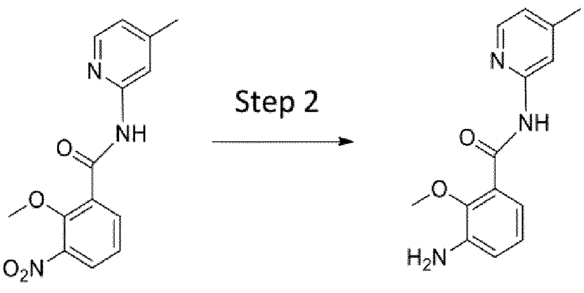


2-Methoxy-N-(4-methylpyrid-2-yl)-3-nitrobenzamide (1.1g, 3.8mmol) and 10% Pd/C (0.15g) were added to 30mL of methanol, and the resulting solution was allowed to react at 25°C under a hydrogen atmosphere until the reaction was completed as detected by TLC. The reaction solution was filtered, and the filtrate was evaporated to dryness to obtain 3-amino-2-methoxy-N-(4-methylpyrid-2-yl)benzamide (0.92g, 93.4%).

MS m/z (ESI): 258.13[M+H]$^+$.

**Step 3**

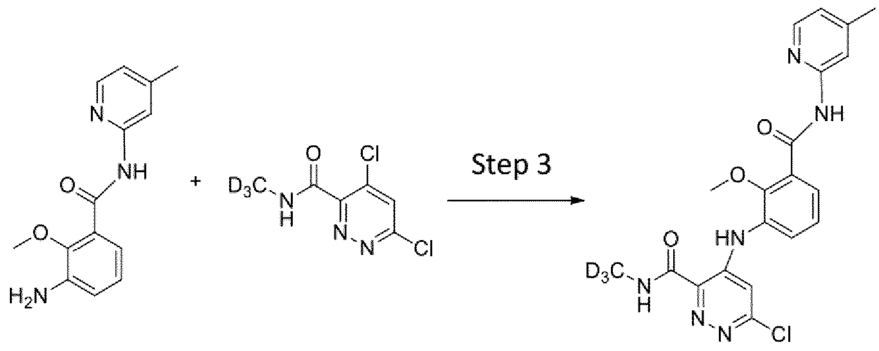


At room temperature, to a solution of 3-amino-2-methoxy-N-(4-methylpyrid-2-yl)benzamide (0.9g, 3.5mmol) and 4,6-dichloro-N-(methyl-d3)pyridazine-3-carboxamide (0.72g, 3.5mmol) in THF (5mL), a solution of bis(trimethylsilyl) amino lithium (1M, 10.5mL, 10.5mmol) in THF was added dropwise. The resulting solution was allowed to react at room temperature until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-chloro-4-((2-methoxy-3-((4-methylpyrid-2-yl)carbamoyl)phenyl)amino)-N-(methyl-d3)pyridazine-3-carboxamide (0.88g, 58%).

MS m/z (ESI): 430.03$[M+H]^+$.

**Step 4**

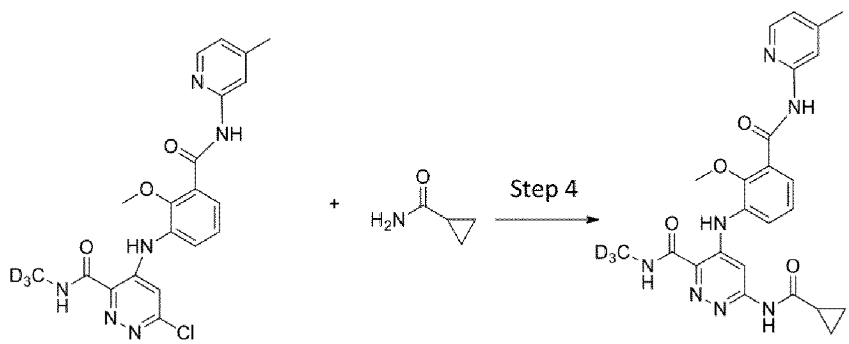


6-Chloro-4-((2-methoxy-3-((4-methylpyrid-2-yl)carbamoyl)phenyl)amino)-N-(methyl-d3)pyridazine-3-carboxamide (0.8g, 1.8mmol), cyclopropanecarboxamide (0.15g, 1.8mmol), tri(dibenzylideneacetone) dipalladium (0.1g, 0.1mmol), 4,5-diphenylphosphine-9,9-dimethyloxaanthracene (0.12g, 0.2mmol) and cesium carbonate (1.27g, 3.9mmol) were added to 1,4-dioxane (10mL), and reacted under reflux with heating until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-(cyclopropanecarboxamido)-4-(2-methoxy-3-(4-methylpyrid-2-yl)carbamoyl)phenyl)amino)-N-(methyl-d3)pyridazine-3-carboxamide (0.42g, 47%).

MS m/z (ESI): 479.24$[M+H]^+$.

$^1$H-NMR(DMSO-$d_6$)$\delta$:11.34(1H,s),10.96(1H,s),10.58(1H,s),9.15(1H,s),8.20-8.08(3H,m),7.62-7.59(1H, m),7.50-7.48(2H,m),7.32-7.29(1H,m),3,75(3H,s),2.30(3H,s),2.07-2.05(1H,m), 0.81-0.79(4H,m).

**Example 38**

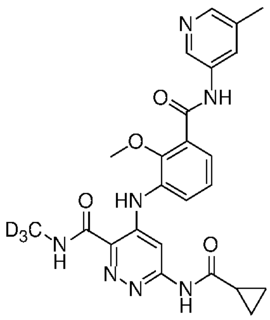

38

With reference to the synthetic method of Example 37, 5-methylpyridine-3-amine was used in place of 4-methylpyridine-2-amine in Step 1, to obtain compound 38.

MS m/z (ESI): 479.24[M+H]$^+$.

$^1$H-NMR(DMSO-$d_6$)δ:11.35(1H,s),10.99(1H,s), 10.51(1H,s), 9.15(1H,s), 8.65(1H,s), 8.15-8.16(2H,m), 8.03(1H,s),7.58-7.61 (1H,m), 7.28-7.37(2H,m), 3.75(3H,s), 2.30(3H,s), 2.07-2.08(1H,m), 0.81-0.83(4H,m).

**Examples 39-52, 105**

With reference to the synthetic method of Example 37, 4-methylpyridine-2-amine in Step 1 was correspondingly replaced to obtain compounds with structures listed in the following table:

| Exam ple No. | Structural formula | m/z [M+H]$^+$ | Examp le No. | Structural formula | m/z [M+H]$^+$ |
|---|---|---|---|---|---|
| 39 | 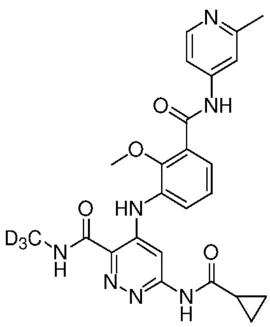 | 479.24 | 40 | 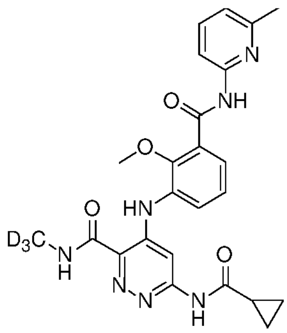 | 479.24 |
| 41 | 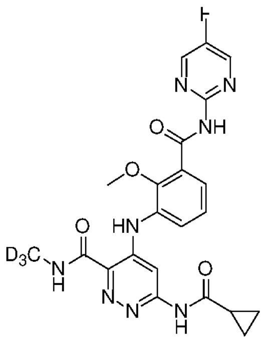 | 484.19 | 42 | 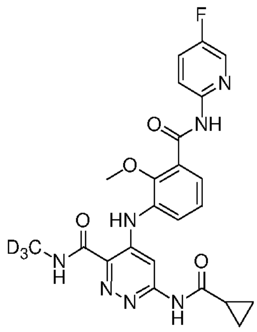 | 483.19 |

(continued)

| Exam ple No. | Structural formula | m/z [M+H]+ | Examp le No. | Structural formula | m/z [M+H]+ |
|---|---|---|---|---|---|
| 43 | 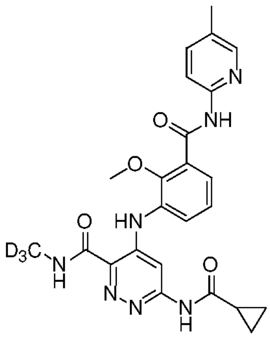 | 479.23 | 44 | 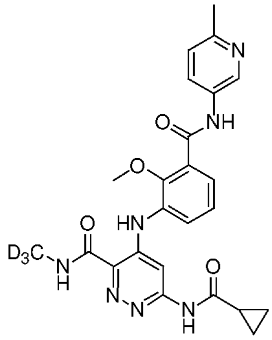 | 479.23 |
| 45 | 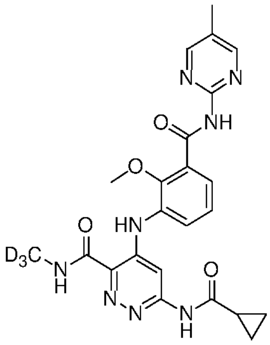 | 480.21 | 46 | 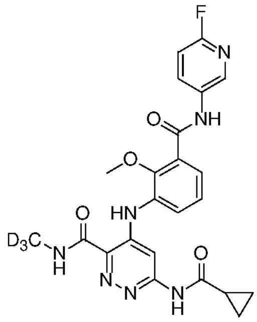 | 483.19 |
| 47 | 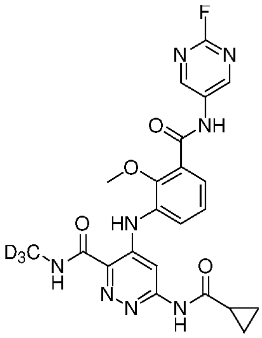 | 484.19 | 48 | 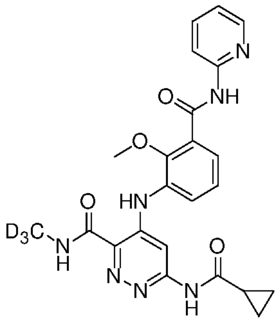 | 465.20 |
| 49 | 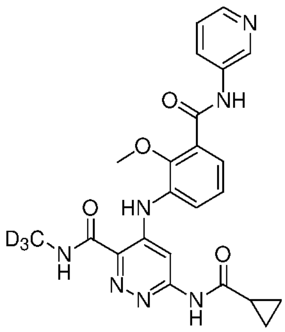 | 465.20 | 50 | 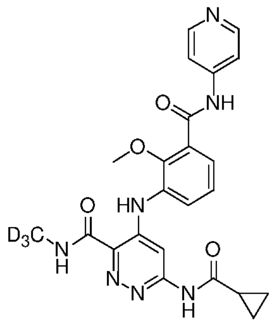 | 465.20 |

(continued)

| Exam ple No. | Structural formula | m/z [M+H]+ | Examp le No. | Structural formula | m/z [M+H]+ |
|---|---|---|---|---|---|
| 51 | 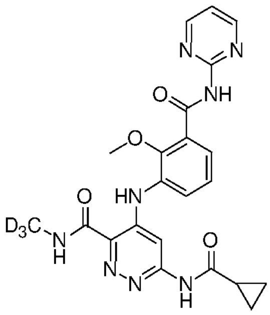 | 466.19 | 52 | 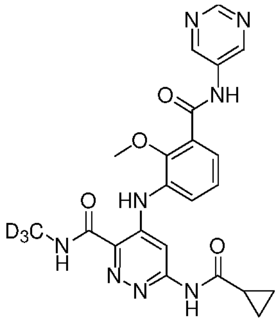 | 466.19 |
| 105 | 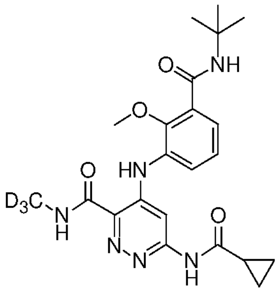 | 444.24 | | | |

**Example 57**

6-(Cyclopropanecarboxamido)-4-((3-((4-fluorophenyl)carbamoyl)-2-methoxyphenyl)amino)-N-(methyl-d3)nicotinamide

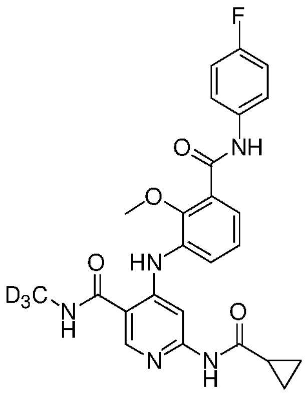

**57**

**Step 1**

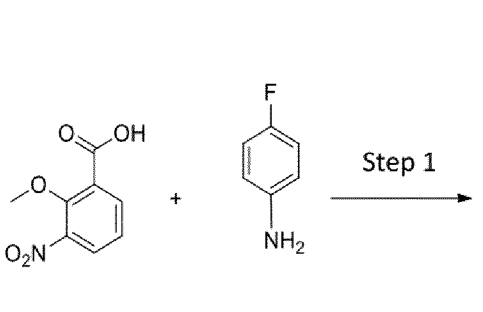


2-Methoxy-3-nitrobenzoic acid (1.0g, 5.1mmol) was added to thionyl chloride (15.0g), and reacted under reflux with stirring and heating until the reaction was completed as detected by TLC. The reaction solution was cooled to room temperature and concentrated under reduced pressure. The residue was dissolved with 5mL of acetone, and added dropwise to a solution of 4-fluoroaniline (0.5g, 4.5mmol) and triethylamine (1.0g, 10.2mmol) in 10mL of acetone, while controlling the temperature to be less than 10°C. After completion of dropping, the resulting solution was allowed to react at room temperature until the reaction was completed as detected by TLC, to which 50mL of water was added, followed by filtering, drying at room temperature for 24h, to obtain N-(4-fluorophenyl)-2-methoxy-3-nitrobenzamide (1.1g, 74.8%).
MS m/z (ESI): 291.38[M+H]$^+$.

**Step 2**

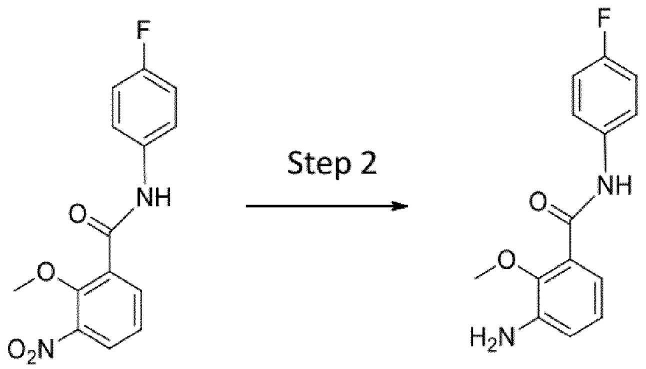


N-(4-fluorophenyl)-2-methoxy-3-nitrobenzamide (1.0g, 3.4mmol) and 10% Pd/C (0.15g) were added to 30mL of methanol, and the resulting solution was allowed to react at 25°C under a hydrogen atmosphere until the reaction was completed as detected by TLC. The reaction solution was filtered, and the filtrate was evaporated to dryness to obtain 3-amino-N-(4-fluorophenyl)-2-methoxybenzamide (0.82g, 91.4%).
MS m/z (ESI): 261.02 [M+H]$^+$.

**Step 3**

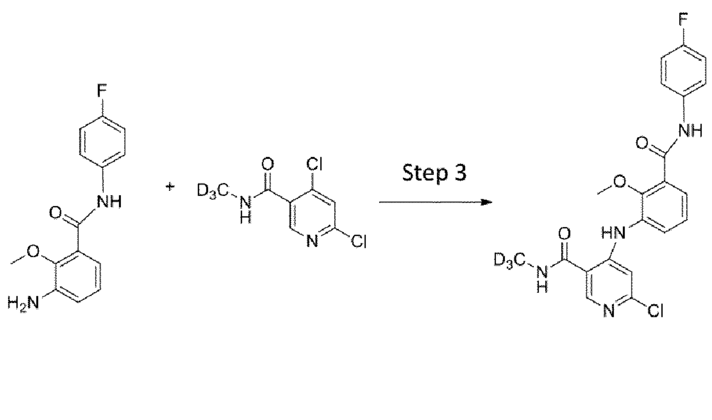

At room temperature, to a solution of 3-amino-N-(4-fluorophenyl)-2-methoxybenzamide (0.8g, 3.1mmol) and 4,6-dichloro-N-(methyl-d3)pyridine-3-carboxamide (0.64g, 3.1mmol) in THF (5mL), a solution of bis(trimethylsilyl)amino lithium (1M, 9.3mL, 9.3mmol) in THF was added dropwise. The resulting solution was allowed to react at room temperature until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-chloro-4-((3-((4-fluorophenyl)carbamoyl)-2-methoxyphenyl)amino)-N-(methyl-d3)nicotinamide (0.92g, 69.3%).
MS m/z (ESI): 432.06[M+H]$^+$.

**Step 4**

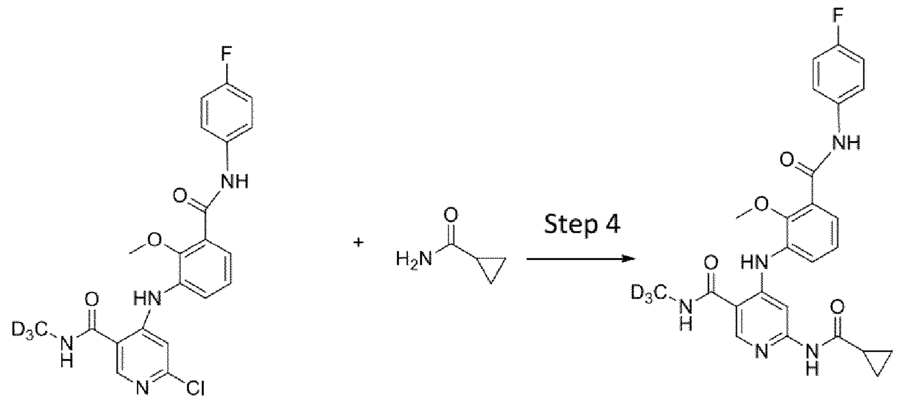


6-Chloro-4-((3-((4-fluorophenyl)carbamoyl)-2-methoxyphenyl)amino)-N-(methyl-d3)nicotinamide (0.9g, 2.0mmol), cyclopropanecarboxamide (0.17g, 2.0mmol), tri(dibenzylideneacetone) dipalladium (0.1g, 0.1mmol), 4,5-diphenylphosphine-9,9-dimethyloxaanthracene (0.12g, 0.2mmol) and cesium carbonate (1.27g, 3.9mmol) were added to 1,4-dioxane (10mL), and reacted under reflux with heating, until the reaction was completed as detected by TLC. The reaction solution was diluted with dichloromethane and washed with saturated sodium chloride aqueous solution. The organic phase was dried over anhydrous magnesium sulfate, concentrated under reduced pressure, and separated by column chromatography to obtain 6-(cyclopropanecarboxamido)-4-((3-((4-fluorophenyl)carbamoyl)-2-methoxyphenyl) amino)-N-(methyl-d3)nicotinamide (0.42g, 42%).
MS m/z (ESI): 481.16[M+H]$^+$.

$^1$H-NMR(DMSO- d6)δ:10.78(1H,s),10.69(1H,s),10.39(1H,s),8.59(1H,s),8.52(1H,s),8.05(1H,s),7.77-7.73(2H, m),7.56-7.53(1H,m),7.27-7.16(4H,m),3.73(3H,s),2.00-1.94(1H,m),0.77(4H,m).
With reference to the synthetic method of Example 57, 4-fluoroaniline in Step 1 was correspondingly replaced to obtain compounds with structures listed in the following table:

| Exam ple No. | Structural formula | m/z [M+H]$^+$ | Examp le No. | Structural formula | m/z [M+H]$^+$ |
|---|---|---|---|---|---|
| 53 | 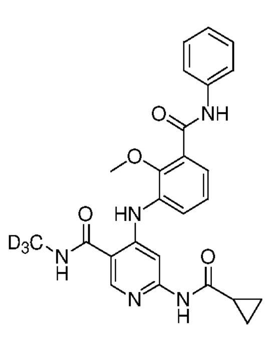 | 463.21 | 54 | 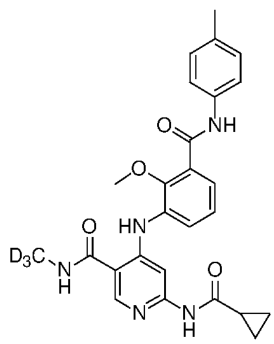 | 477.22 |

(continued)

| Example No. | Structural formula | m/z [M+H]+ | Example No. | Structural formula | m/z [M+H]+ |
|---|---|---|---|---|---|
| 55 | 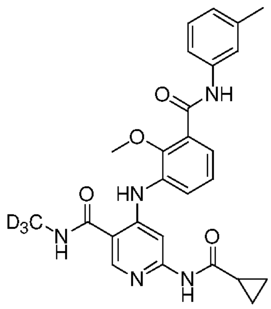 | 477.22 | 56 | 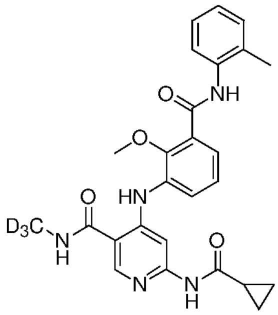 | 477.22 |
| 58 | 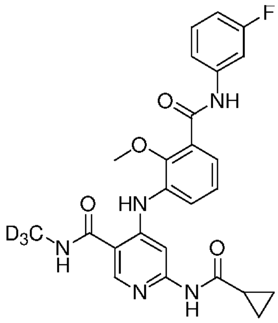 | 481.16 | 59 | 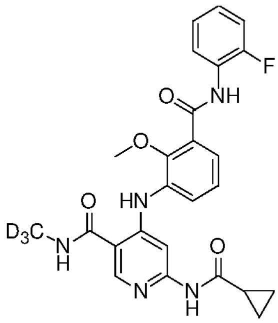 | 481.16 |
| 60 | 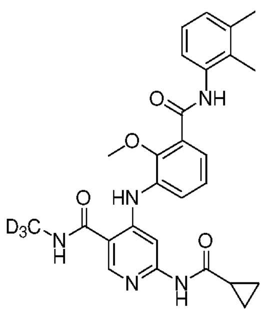 | 491.24 | 61 | 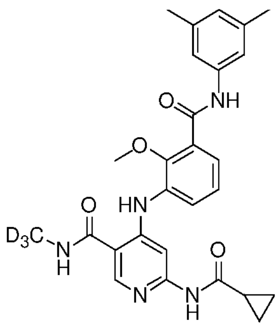 | 491.24 |
| 62 | 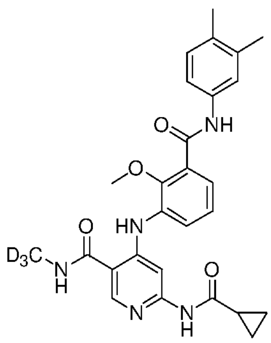 | 491.24 | 63 | 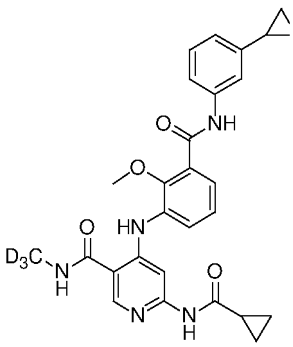 | 503.24 |

(continued)

| Example No. | Structural formula | m/z [M+H]+ | Example No. | Structural formula | m/z [M+H]+ |
|---|---|---|---|---|---|
| 64 | 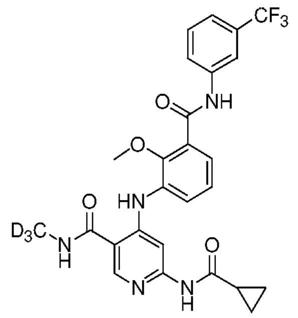 | 531.20 | 65 | 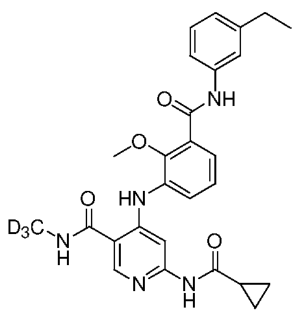 | 491.24 |
| 66 | 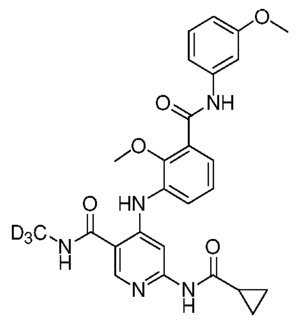 | 493.22 | 67 | 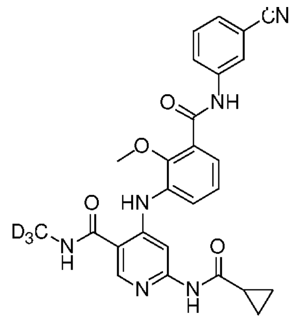 | 488.20 |
| 68 | 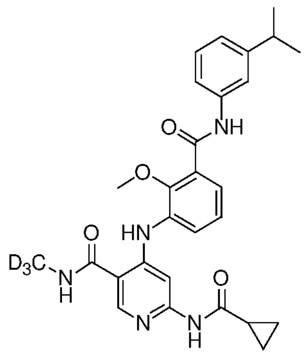 | 505.26 | 69 | 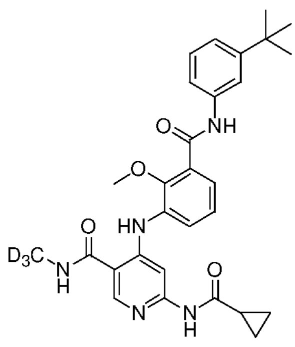 | 519.27 |
| 70 | 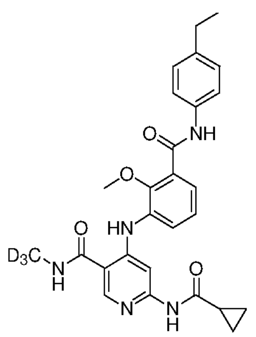 | 491.24 | 71 | 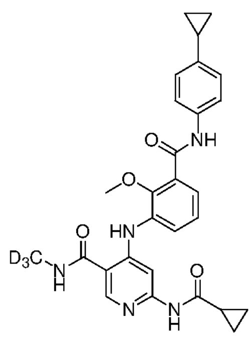 | 503.24 |

(continued)

| Exam ple No. | Structural formula | m/z $[M+H]^+$ | Examp le No. | Structural formula | m/z $[M+H]^+$ |
|---|---|---|---|---|---|
| 72 | 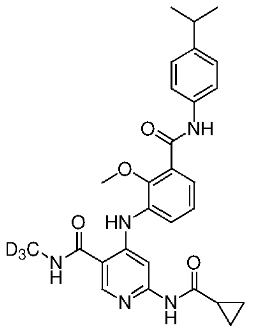 | 505.26 | 73 | 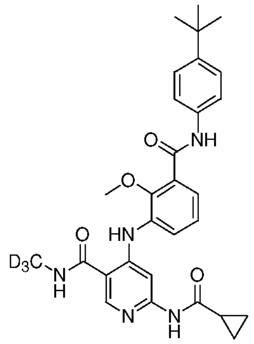 | 519.27 |
| 74 | 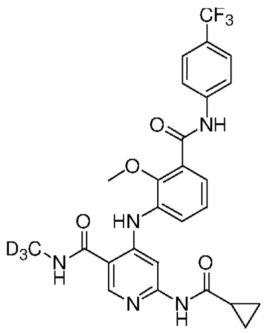 | 531.20 | 75 | 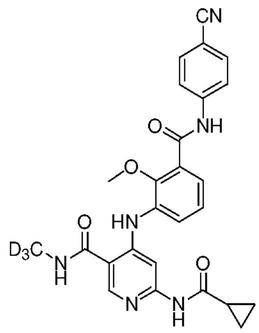 | 488.20 |
| 76 | 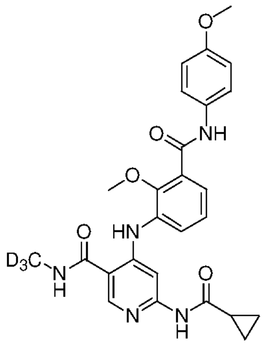 | 493.22 | 77 | 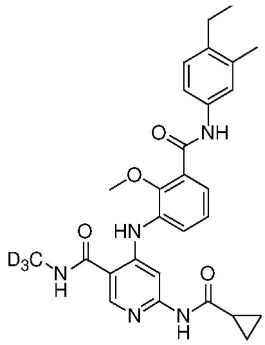 | 505.26 |
| 78 | 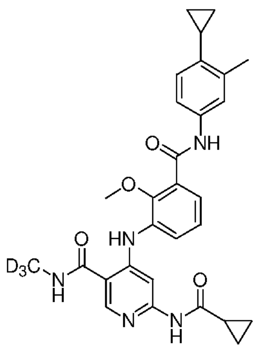 | 517.26 | 79 | 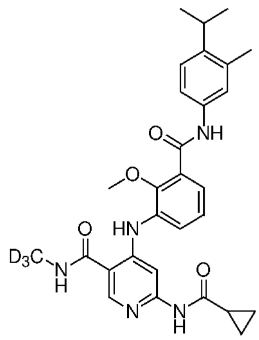 | 519.27 |

(continued)

| Example No. | Structural formula | m/z [M+H]+ | Examp le No. | Structural formula | m/z [M+H]+ |
|---|---|---|---|---|---|
| 80 | 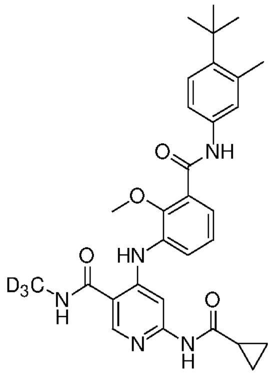 | 533.29 | 81 | 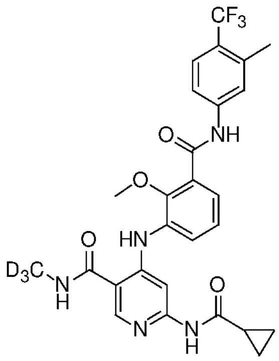 | 545.21 |
| 82 | 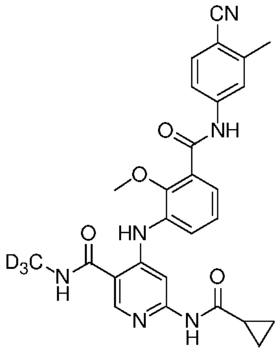 | 502.22 | 83 | 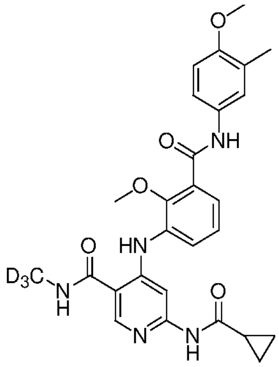 | 507.24 |
| 84 | 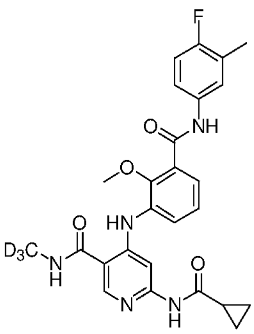 | 495.22 | 85 | 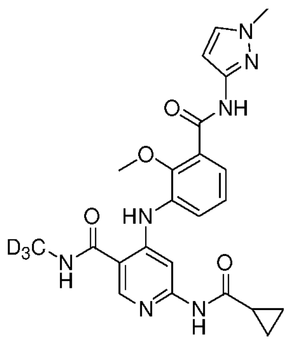 | 467.22 |
| 86 | 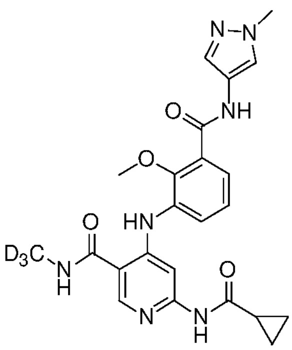 | 467.22 | 87 | 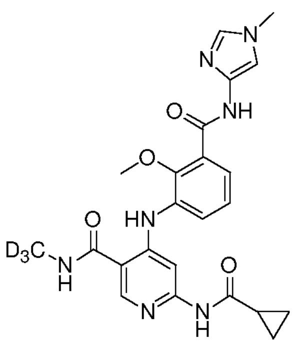 | 467.21 |

(continued)

| Example No. | Structural formula | m/z [M+H]+ | Example No. | Structural formula | m/z [M+H]+ |
|---|---|---|---|---|---|
| 88 | 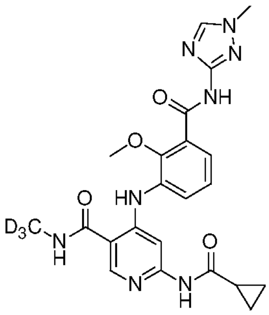 | 468.21 | 89 | 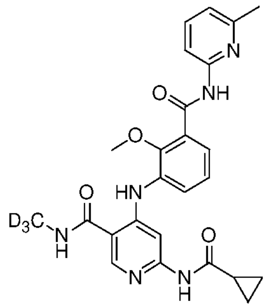 | 478.22 |
| 90 | 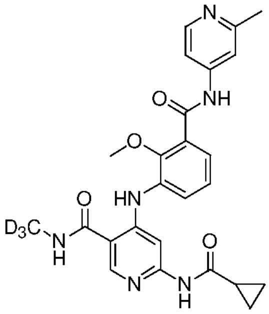 | 478.22 | 91 | 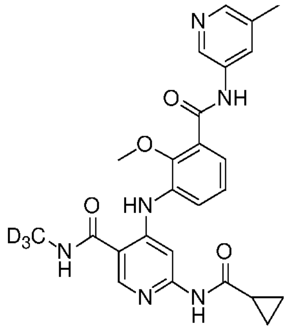 | 478.23 |
| 92 | 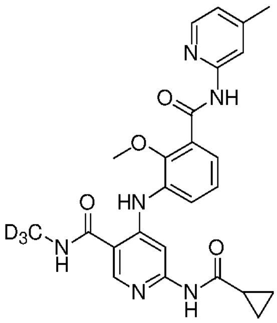 | 478.17 | 93 | 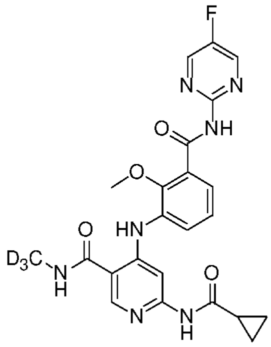 | 483.19 |
| 94 | 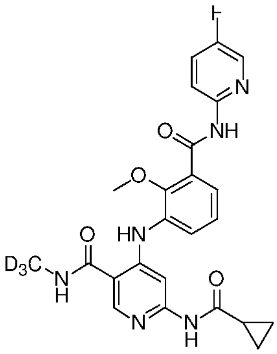 | 482.20 | 95 | 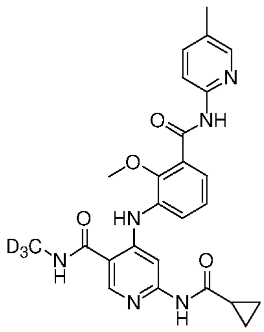 | 478.18 |

(continued)

| Example No. | Structural formula | m/z [M+H]+ | Example No. | Structural formula | m/z [M+H]+ |
|---|---|---|---|---|---|
| 96 | 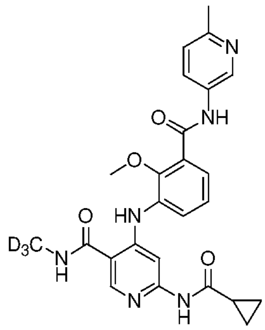 | 478.22 | 97 | 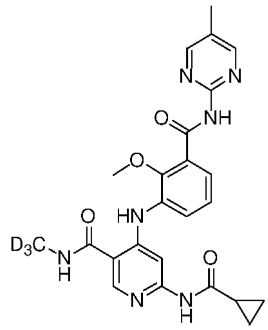 | 479.22 |
| 98 | 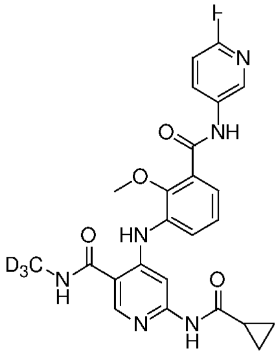 | 482.20 | 99 | 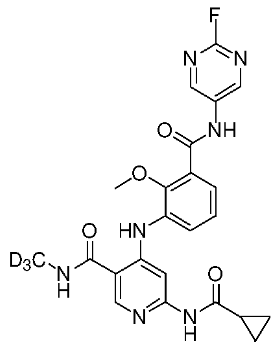 | 483.19 |
| 100 | 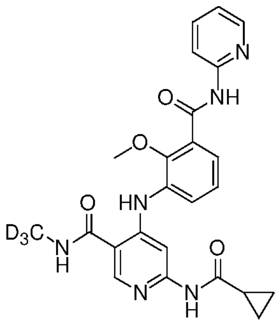 | 464.20 | 101 | 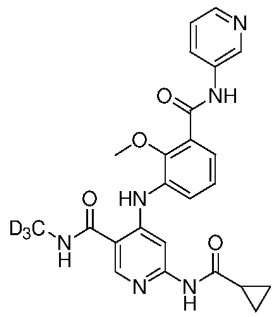 | 464.20 |
| 102 | 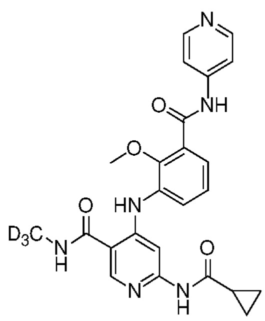 | 464.20 | 103 | 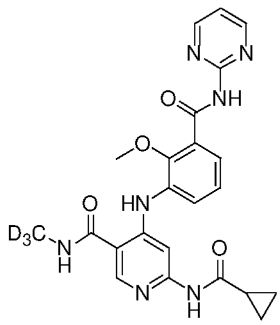 | 465.20 |

(continued)

| Example No. | Structural formula | m/z $[M+H]^+$ | Example No. | Structural formula | m/z $[M+H]^+$ |
|---|---|---|---|---|---|
| 104 | 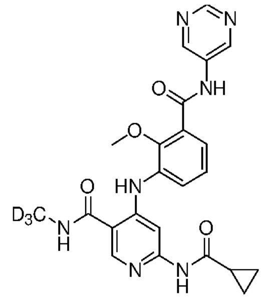 | 465.20 | 106 | 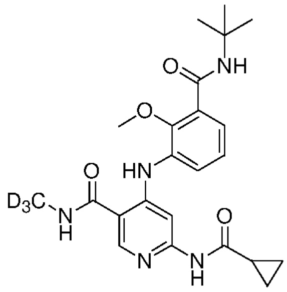 | 443.24 |

BMS-986165 was prepared in accordance with CN201380069692.9, with the specific structure:

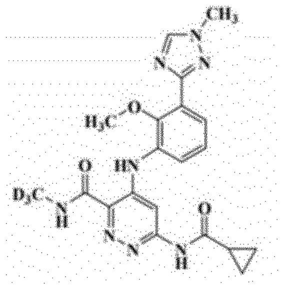

.

**Biological assay**

The present invention is further described and explained below in connection with the test examples, but these examples are not meant to limit the scope of the present invention.

**Test example 1: In Vitro Cellular Inhibition Assay**

The inhibitory effect of the compounds of formula (I) according to the present invention on the TYK2 signaling pathway in cells was assessed using the following method:
Instrumentation: SpectraMax Paradigm reader.

Method: in this experiment, a Ba/F3 cell line expressing TYK2 was employed to assess the effect of the compounds on cell proliferation inhibition by measuring their effects on cell activity in vitro.

Procedure: a CellTiter-Glo buffer was thawed and placed aside till room temperature. A CellTiter-Glo substrate was placed aside till room temperature and dissolved with the buffer to prepare a CellTiter-Glo working solution. To a 96-well flat bottom transparent drug plate, 98 μL of cell culture medium and 2 μL of gradient diluted compound solution were added. The drug plate was placed aside for 10 min, and then 50 μL of the CellTiter-Glo working solution was added. The drug plate was subsequently shaken on an orbital shaker for 2 min and placed aside at room temperature for an additional 10 min. Finally, the luminescence signal was measured on the SpectraMax Paradigm reader.

Data Analysis: the cell proliferation inhibition rate data were processed using the following formula:

$$\text{Inhibition Rate (Inh\%)} = 100\text{-(RLUDrug-RLUMin)/(RLUMax-RLUMin)} \times 100\%.$$

The inhibition rates of compounds with different concentrations were calculated in EXCEL, and then GraphPad Prism software was employed to plot inhibition rate curve and calculate relevant parameters, including maximum and minimum cell inhibition rates, $IC_{50}$ values.

The inhibitory activity of the compounds obtained by the aforementioned scheme was as follows:

| Example No. | $IC_{50}$ (nM) | Example No. | $IC_{50}$ (nM) | Example No. | $IC_{50}$ (nM) |
|---|---|---|---|---|---|
| 1 | B | 23 | B | 45 | B |

(continued)

| Example No. | $IC_{50}$ (nM) | Example No. | $IC_{50}$ (nM) | Example No. | $IC_{50}$ (nM) |
|---|---|---|---|---|---|
| 2 | A | 24 | B | 46 | B |
| 3 | A | 25 | B | 47 | B |
| 4 | A | 26 | B | 48 | B |
| 5 | A | 27 | B | 49 | B |
| 6 | B | 28 | B | 50 | B |
| 7 | B | 29 | B | 51 | B |
| 8 | B | 30 | B | 52 | B |
| 9 | B | 31 | B | 53 | B |
| 10 | B | 32 | B | 54 | A |
| 11 | B | 33 | B | 55 | A |
| 12 | B | 34 | A | 56 | B |
| 13 | B | 35 | B | 57 | A |
| 14 | B | 36 | B | 58 | B |
| 15 | B | 37 | B | 59 | B |
| 16 | B | 38 | A | 60 | B |
| 17 | B | 39 | B | 61 | B |
| 18 | B | 40 | B | 62 | A |
| 19 | B | 41 | B | 63 | B |
| 20 | B | 42 | B | 64 | B |
| 21 | B | 43 | B | 65 | B |
| 22 | B | 44 | B | 66 | B |
| 67 | B | 81 | B | 95 | B |
| 68 | B | 82 | B | 96 | B |
| 69 | B | 83 | B | 97 | B |
| 70 | B | 84 | B | 98 | B |
| 71 | B | 85 | B | 99 | B |
| 72 | B | 86 | A | 100 | B |
| 73 | B | 87 | B | 101 | B |
| 74 | B | 88 | B | 102 | B |
| 75 | B | 89 | B | 103 | B |
| 76 | B | 90 | B | 104 | B |
| 77 | B | 91 | A | 105 | B |
| 78 | B | 92 | B | 106 | B |
| 79 | B | 93 | B | | |
| 80 | B | 94 | B | | |
| Notes: A denotes 1nM < $IC_{50}$ < 5nM, B denotes 5nM < $IC_{50}$ < 10nM. | | | | | |

Conclusion: the compounds according to the present invention have a significant inhibitory effect on the TYK2 signaling pathway in cells.

**Test example 2: hERG Potassium Channel Inhibition Assay**

Instrumentation: HEKA EPC 10 patch clamp amplifier.

Method: in this experiment, the blocking effect of the compounds on the current of the HEK-293 cell line stably expressing hERG channel was measured using the manual patch clamp technique, and the risk of the compounds on the inhibition of cardiac hERG potassium channel was assessed by fitting the concentration effect relationship.

Instrumentation: SpectraMax Paradigm reader.

Method: in this experiment, expressing cell lines were employed to assess the cardiac toxicity of the compounds by measuring their inhibitory effects on cell activity in vitro.

Procedure: in the patch clamp operation, firstly, a capillary glass tube was pulled into a recording electrode using a microelectrode puller, then an electrode filled with intracellular solution was mounted into a microelectrode holder. A microelectrode manipulator was operated under an inverted microscope to immerse the electrode into an extracellular solution and the electrode resistance (Rpip) was recorded. Then, the electrode was slowly brought into contact with the cell surface, and a negative pressure was applied for suction to form a GΩ seal. At this point, a fast capacitance compensation was performed, and a negative pressure was continuously applied to suck and break the cell membrane to form a whole cell recording mode. Finally, a slow capacitance compensation was performed and experimental parameters including series resistance (Rs) and the like were recorded. No leakage compensation was performed. When the hERG current recorded by the whole cell was stable, the administration of drugs commenced, and each drug concentration acted for about 5 min (or until the current stabilization) before detecting the next concentration. A cover glass covered with cells was placed in a recording bath under an inverted microscope, the blank control extracellular solution and the working solution of the compounds to be tested sequentially flowed through the recording bath from low concentration to high concentration by using gravity perfusion to act on the cells, and solution exchange was performed using a peristaltic pump during recording. The current measured by each cell in the extracellular solution without the compound served as its own control group. Each concentration was independently assessed twice. All electrophysiological tests were performed at room temperature.

Data Analysis: firstly, the tail current ($\textit{Peak tail current}_{compound}$) after the action of each drug concentration and the blank control tail current ($\textit{Peak tail current}_{control}$) were normalized, then the inhibition rate ( $1\frac{Peak\ tail\ current_{compound}}{Peak\ tail\ current_{control}}$ ) corresponding to each drug concentration was calculated, and the mean (Mean), standard deviation (SD) and standard error (SE) of the inhibition rate corresponding to each concentration were calculated, with data expressed as Mean ± SE. The calculation of $IC_{50}$ value and the fitting of dose-response curve for the compound were performed using GraphPad Prism software.

Conclusion: the hERG detection of compounds such as Examples 2, 3, 4, 5, 34, 38, 54, 55, 57, 62, 86, 91 according to the present invention show no significant inhibition (negative results) at the tested concentrations, indicating a lower potential for cardiac toxicity related to hERG blockade..

**Test example 3: Kinase Selectivity Profiling**

**3.1 JAK1-3 /TYK2 JH1 enzyme binding assay in vitro**

Instrumentation: Envision microplate reader (PerkinElmer), ECHO550 (LABCYTE)

Method: in this experiment, the inhibitory effects of compounds on JAK1-3/TYK2 JH1 kinase was tested using time-resolved fluorescence resonance energy transfer (TR-FRET) method.

Procedure: compounds were prepared into 10 mM stock solutions using DMSO, which were further diluted into compound dilutions with different concentration gradients in DMSO, and the 100× compound dilutions were transferred to a 384-well assay plate using an Echo instrument. In the experiment, Assay buffer, SEB, TK-Substrate-biotin, detection reagent, and the like were all sourced from TK kit (Cisbio, Cat # 62TK0PEC). The preparation of the following three 1× assay working solutions: 1.Working solution for JAK1 JH1 assay: Assay buffer at a final concentration of 1×, $MgCl_2$ at a final concentration of 5 mM, EGTA at a final concentration of 0.625 mM, SEB at a final concentration of 60 nM, Brij-35 at a final concentration of 0.01%, DTT at a final concentration of 1 mM, 2. Working solution for JAK2-3 JH1 assay: Assay buffer at a final concentration of 1×, $MgCl_2$ at a final concentration of 5 mM, DTT at a final concentration of 1 mM, 3. Working solution for TYK2 JH1 assay: Assay buffer at a final concentration of 1×, $MgCl_2$ at a final concentration of 5 mM, $MnCl_2$ at a final concentration of 1 mM, SEB at a final concentration of 12.5 nM, DTT at a final concentration of 1 mM. JAK1-3/TYK2 JH1 kinase with a 2× final concentration and 2× TK-Substrate-biotin substrate were prepared using their respective 1× working solution. 5uL of JAK1-3/TYK2 JH1 kinase was added to a 384-well assay plate, and centrifuged at 1000rpm for 30 s, followed by incubation at room temperature for 15 min. 5uL of TK-Substrate-biotin substrate was added to the 384-well assay plate, and centrifuged at 1000rpm for 30 s, followed by incubation at room temperature for 45 min for JAK1-2 JH1 and 60 min for JAK3/TYK2 JH1. 10uL of prepared 2× detection reagent was added to the 384-well assay plate, and

centrifuged at 1000rpm for 30 s, followed by incubation at room temperature for 60 min for JAK1-2 JH1 and 120 min for JAK3/TYK2 JH1, and then incubation at 4°C overnight. Finally, the 665nm/615nm fluorescence signal ratio was measured using the Envision microplate reader (PerkinElmer).

Data Analysis: XLfit, a software developed by IDBS and integrated into the Microsoft Excel environment, was used to process and analyze the test data. Firstly, the average reaction signals of high signal control wells and low signal control wells were calculated separately, and then the reaction inhibition rate of each compound well was calculated according to the formula "single well inhibition rate% = 100% - (average value of high signal control group - single well signal value)/(average value of high signal control group - average value of low signal control group) × 100%". Thereafter, the concentration and corresponding inhibition rate data were imported into the XLfit software, and the Dose Response One Site 205 model within the software was employed to fit the inhibition rate-concentration curve by four-parameter method, and the $IC_{50}$ value of the compound was calculated.

### 3.2 JAK1 JH2 enzyme binding assay in vitro

Instrumentation: Envision microplate reader (PerkinElmer), Echo (LABCYTE)

Method: in this experiment, the inhibitory effects of compounds on JAK1 JH2 pseudokinase was tested using time-resolved fluorescence resonance energy transfer (TR-FRET) method.

Procedure: compounds were prepared into 10 mM stock solutions using DMSO, which were further diluted into compound dilutions with different concentration gradients in DMSO, and the compound dilutions with 200× final concentration were transferred to a 384-well assay plate using an Echo instrument. The preparation of the following 1× assay working solution: HEPES pH7.5 at a final concentration of 20mM, $MgCl_2$ at a final concentration of 10mM, Brij-35 at a final concentration of 0.015%, DTT at a final concentration of 2 mM, BSA at a final concentration of 50ug/mL. JAK1 JH2 pseudokinase with 3× final concentration, Tb antibody and Tracer were prepared using their respective 1× working solution. 5uL of JAK1 JH2 pseudokinase was added to a 384-well assay plate, and centrifuged at 1000rpm for 30 s. 5uL of Tb antibody was added to the 384-well assay plate, and centrifuged at 1000rpm for 30 s. 5uL of Tracer was added to the 384-well assay plate, and centrifuged at 1000rpm for 30 s. Incubation was performed at room temperature for 60 min, and then at 4°C overnight. Finally, the 495nm/520nm fluorescence signal ratio was measured using the Envision microplate reader (PerkinElmer).

Data Analysis: XLfit, a software developed by IDBS and integrated into the Microsoft Excel environment, was used to process and analyze the test data. Firstly, the average reaction signals of high signal control wells and low signal control wells were calculated separately, and then the reaction inhibition rate of each compound well was calculated according to the formula "single well inhibition rate% = 100% - (average value of high signal control group - single well signal value)/(average value of high signal control group - average value of low signal control group) × 100%". Thereafter, the concentration and corresponding inhibition rate data were imported into the XLfit software, and the Dose Response One Site 205 model within the software was employed to fit the inhibition rate-concentration curve by four-parameter method, and the $IC_{50}$ value of the compound was calculated.

### 3.3 TYK2 JH2 enzyme binding assay in vitro

Instrumentation: Envision microplate reader (PerkinElmer), ECHO550 (LABCYTE).

Method: the inhibitory effects of compounds on TYK2 JH2 pseudokinase was tested using time-resolved fluorescence resonance energy transfer (TR-FRET) method.

Procedure: compounds were prepared into 10 mM stock solutions using DMSO, which were further diluted into compound dilutions (200×) with different concentration gradients in DMSO, and the compound dilutions (200×) were transferred to a 384-well assay plate using an Echo instrument. Dilution buffer (20 mM HEPES pH 7.5, 10 mM $MgCl_2$, 0.015% Brij-35, 2 mM DTT and 50 μg/mL BSA) was prepared. TYK2 JH2 pseudokinase, Tb antibody, and Tracer working solutions were prepared using the dilution buffer, respectively. 5uL of TYK2 JH2 pseudokinase working solution (final concentration 0.5 nM) was added to a 384-well assay plate, and centrifuged at 1000rpm for 30 s. 5uL of Tb antibody (final concentration 1×) was added to the 384-well assay plate, and centrifuged at 1000rpm for 30 s. 5uL of Tracer (final concentration 0.5 nM) was added to the 384-well assay plate, and centrifuged at 1000rpm for 30 s. Incubation was performed at room temperature for 60 min, and then at 4°C overnight. Finally, the 495nm/520nm fluorescence signal ratio was measured using the Envision microplate reader (PerkinElmer).

Data Analysis: XLfit, a software developed by IDBS and integrated into the Microsoft Excel environment, was used to process and analyze the test data. Firstly, the average reaction signals of high signal control group and low signal control group were calculated separately, and then the reaction inhibition rate of each compound well was calculated according to the formula "single well inhibition rate% = 100% - (average value of high signal control group - single well signal value)/(average value of high signal control group - average value of low signal control group) × 100%". Thereafter, the concentration and corresponding inhibition rate data were imported into the XLfit software, and the Dose Response

One Site 205 model within software was employed to fit the inhibition rate-concentration curve by four-parameter method, and the $IC_{50}$ value of the compound was calculated.

| Example No. | TYK2 JH2 $IC_{50}$ (nM) | JAK 1 JH2 $IC_{50}$ (nM) | JAK 1 JH1 $IC_{50}$ (nM) | JAK 2 JH1 $IC_{50}$ (nM) | JAK 3 JH1 $IC_{50}$ (nM) | TYK2 JH1 $IC_{50}$ (nM) |
|---|---|---|---|---|---|---|
| **2** | 0.311 | 6.565 | >10000 | >10000 | >10000 | >10000 |
| **3** | 0.274 | 9.736 | >10000 | >10000 | >10000 | >10000 |
| **4** | 0.293 | 5.327 | >10000 | >10000 | >10000 | >10000 |
| **5** | 0.281 | 6.933 | >10000 | >10000 | >10000 | >10000 |
| **34** | 0.341 | 4.161 | >10000 | >10000 | >10000 | >10000 |
| **38** | 0.322 | 2.355 | >10000 | >10000 | >10000 | >10000 |
| **54** | 0.302 | 4.565 | >10000 | >10000 | >10000 | >10000 |
| **55** | 0.298 | 3.299 | >10000 | >10000 | >10000 | >10000 |
| **57** | 0.284 | 5.677 | >10000 | >10000 | >10000 | >10000 |
| **62** | 0.277 | 14.32 | >10000 | >10000 | >10000 | >10000 |
| **86** | 0.299 | 17.61 | >10000 | >10000 | >10000 | >10000 |
| **91** | 0.312 | 3.294 | >10000 | >10000 | >10000 | >10000 |
| BMS-986165 | 0.451 | 0.871 | >10000 | >10000 | >10000 | >10000 |

Conclusion: the selectivity level of the compounds according to the present invention towards JH1 target of JAK1~3/TYK2 is comparable to that of BMS-986165, with neither exhibiting inhibitory activity. In addition, the inhibitory activity of them towards JAK1 JH2 is significantly reduced compared to that of BMS-986165. The compounds according to the present invention have favorable selectivity towards TYK2 JH2 and low off-target effects.

**Test example 4: In Vivo Efficacy: Imiquimod-Induced Mouse Psoriasis-Like Model**

The efficacy of the compounds according to the present invention in an imiquimod-induced mouse psoriasis-like model could be assessed using the following method:
Sample preparation: with reference to the preparation of commercially available Benvitimod cream. The compound (the compound of Example 3), the oil phase (hexadecanol, vaseline, liquid paraffin, and glyceryl mono- and distearate), the aqueous phase (propylene glycol, Tween 80, and water), and the appropriate additive (ethylparaben) were mixed, and subjected to vacuum emulsification to prepare a 1% cream sample.

A test site on the back of the animal was shaved to expose an area measuring of $2 \times 3$ $cm^2$. Imiquimod ointment was continuously applied to the back skin of the mice for 6 days to establish a mouse psoriasis model. From day 1 to day 6, various groups of animals were administered respectively according to the experimental protocol. The experimental design of the imiquimod-induced mouse psoriasis-like model was shown in the following table:

| Group | Drug | Administration dose (mg/kg) or administration concentration and quantity | Administration mode/frequenc y | Number of samples per group |
|---|---|---|---|---|
| Blank control group | Blank matrix | / | topical, qd | 10 |
| Model group | Blank matrix | / | topical, qd | 10 |
| Benvitimod cream group (commercially available) | Benvitimod cream | 1%, 100 mg | topical, bid | 10 |
| BMS-986165 group | BMS-986165 | 10 mg/kg | oral, bid | 10 |
| Example 3 compound group | Compound 3 | 1%, 100 mg | topical, qd | 10 |

At day 7, the severity of skin inflammation was asssessed and scored using a 5-point scale (0-4) (PASI score):

Skin thickness: 0: the skin was smooth without wrinkles; 1: the skin at the edge of the drug application area had slight wrinkles; 2: the skin in the drug application area all had slight wrinkles; 3: the degree of wrinkles in the drug application area was further deepened; and 4: on the basis of the score of 3, the mouse had the conditions of weight loss or poor state and the like.

Scabbing: 0: the skin was smooth without scales; 1: the skin in the drug application area had slight scales; 2: the skin in the drug application area was fully covered by scales; 3: the degree of scales in the drug application area was further deepened; and 4: on the basis of the score of 3, the mouse had the conditions of weight loss or poor state and the like.

Erythema: 0: the skin was smooth; 1: the skin in the drug application area was slightly red; 2: the skin in the drug application area was completely red; 3: the degree of red in the drug application area was further deepened; and 4: on the basis of the score of 3, the mouse had the conditions of weight loss or poor state and the like.

The comparison of PASI scores of different compounds in the imiquimod-induced mouse psoriasis-like model was shown in the following table:

| Group | Percentage reduction in PASI score % |
|---|---|
| Model group | / |
| Benvitimod cream group (commercially available) | 8.1 |
| BMS-986165 group | 30.1** |
| Example 3 compound group | 40.7*** |

Notes: compared to the model group, ****P*<0.001, **P*<0.01, the data representing the mean PASI score within the group.

Conclusion: the compound of Example 3 effectively improved psoriasis symptoms in the imiquimod-induced mouse psoriasis-like model, with a significant difference (P<0.001) compared with the model group, and demonstrated superior efficacy compared to Benvitimod cream and BMS-986165. The compounds of Examples 2, 4, 5, 34, 38, 54, 55, 57, 62, 86, 91, etc., showed similar pharmacological effects to the compound of Example 3, and thus were not elaborated on here for brevity.

In the imiquimod-induced mouse psoriasis-like model, the compounds of Examples 2, 3, 4, 5, 34, 38, 54, 55, 57, 62, 86, 91 and the like can effectively improve psoriasis symptoms by oral administration (oral gavage, 10 mg/kg, 25 mg/kg, 50 mg/kg, bid), with significant differences compared with the model group.

The pharmacodynamic experiments showed that there were some differences in the efficacy of anti-psoriasis when the same compound was administered via oral or topical routes.

**Test example 5: In vivo- Mouse Asthma Model**

The efficacy of the compounds according to the present invention in a mouse asthma model induced by OVA could be assessed using the following method:

5.1 Preparation method of test sample

Test sample: the compound of Example 3 was weighed accurately, and dissolved in DMSO to prepare a solution at a concentration of 2mg/mL.

5.2 Preparation of model

30 female BALB/c mice were adaptively fed. After one week, 10 mice were randomly taken out and fed as a blank control group, and 20 mice were selected to establish the asthma model. The model mice were intraperitoneally injected with 0.2mL of sensitizing solution for sensitization respectively on 0, 7 and 14 days. The sensitization solution contained 50 μg of OVA and 2 mg of $Al(OH)_3$. The blank control group received an equivalent volume of physiological saline.

From day 21 to day 23 after sensitization, the mice in the model group and the administration group were subjected to nebulization stimulation with 5% OVA for 30 min, and the mice in the blank control group received nebulization

with physiological saline for the same duration.

5.3 Group Assignment and Dosing

30 min before daily stimulation of the mice, 50 μl of physiological saline was administered endotracheally to mice in the blank control and model groups, and 50 μl of the corresponding drug was administered endotracheally to mice in the Example 3 compound group, once daily for 3 consecutive days. The animal grouping and administration dose were shown in the following table:

| Group | Drug concentration (mg/mL) | Administration volume (μL) | Number of animals (individual) |
|---|---|---|---|
| Blank control | - | 50 | 10 |
| Model group | - | 50 | 10 |
| Example 3 compound group | 2.0 | 50 | 10 |

5.4 Airway Hyperresponsiveness (AHR) Measurement

Airway hyperresponsiveness was measured in each group of mice after the last stimulation. The mice were placed in a whole-body plethysmograph. After recording the baseline values of enhanced pause (Penh), the mice were stimulated by nebulized methacholine, wherein the concentrations of the methacholine were 0, 6.25 and 12.5mg/mL from low to high , the nebulization volume was 100 μL per dose for 60 s. After completion of each concentration of nebulization, whether the mice exhibited hypoxia such as dyspnea, head scratching and restlessness was observed, then the Penh values were recorded for 3 min, and the mean values were used to compare the AHR in each group of mice.

5.5 Detection of inflammatory cells in bronchoalveolar lavage fluid

After the airway hyperresponsiveness measurement, the mice were euthanized by cervical dislocation and fixed on a dissecting tray. The abdomens and thoracic cavities of the mice were opened, and the neck skin and excess tissues were excised to expose the mouse trachea. A small incision was made on the transverse axis of the trachea with small scissors, a pre-prepared 1mL syringe needle was inserted therein, and the needle was fixed with surgical thread. 0.5 mL of pre-cooled phosphate-buffer solution (PBS) was aspirated with a 1 mL syringe and slowly injected into the lungs of the mice, followed by slow withdrawal. Each mouse was lavaged once, and a total of about 0.4 mL of lavage fluid was collected. The number of inflammatory cells in the bronchoalveolar lavage fluid was measured by a cell counter.

5.6 Statistical Analysis

Statistical analysis was conducted using SPSS software, Measurement data were expressed as mean ± standard deviation. One Way ANOVA was used for inter-group comparisons, LSD test was used for those with homogeneous variance, and Dunnett-t test was used for those with heterogeneous variance. Differences were statistically significant with $P<0.05$.

5.7 Results

5.7.1 Effect on the enhanced pause (Penh) value of the mice

The results were as follows:

| Group | Concentration of methacholine (mg/mL) | | |
|---|---|---|---|
| | 0 | 6.25 | 12.5 |
| Blank control group | 0.88±0.16 | 1.27±0.12 | 2.70±0.79 |
| Model group | 0.83±0.20 | 2.34±0.36$^{###}$ | 3.65±0.32$^{#}$ |

(continued)

| Group | Concentration of methacholine (mg/mL) | | |
|---|---|---|---|
| | 0 | 6.25 | 12.5 |
| Compound of Example 3 | 0.86±0.31 | 1.79±0.45* | 2.94±0.66* |
| Notes: compared to the blank control group, #*P*<0.05, ###*P*<0.001; compared to the model group, **P*<0.05. | | | |

Mice were sensitized with OVA+$Al(OH)_3$ for 3 weeks, followed by continuous stimulation with 5% OVA for 3 days. After subsequent stimulations with methacholine at concentrations of 6.25 mg/mL and 12.5 mg/mL, compared with the blank control group, the Penh value exhibited a significant increase (P<0.001 or P<0.05), indicating successful establishment of the mouse asthma model.

Compared to the model group, the administration of the compound of Example 3 to the mice before stimulation could significantly reduce their enhanced pause (Penh) values *(P<0.05).*

5.7.2 Effect on inflammatory cells in bronchoalveolar lavage fluid of the mice

The results were as follows:

| Group | Number of inflammatory cells (×$10^3$) | | | |
|---|---|---|---|---|
| | Leukocyte | Lymphocyte | Monocyte | Neutrophil |
| Blank control group | 365.7±270.7 | 207.6±167.0 | 9.5±10.1 | 148.6±104.5 |
| Model group | 2,348.9±895.4### | 1,717.8±849.7### | 77.8±46.5## | 553.3±281.6## |
| Example 3 compound group | 1,241.7±618.1* | 791.7±524.9* | 58.3±37.0 | 391.7±206.3 |
| Notes: compared to the blank control group, ##*P*<0.01, ###*P*<0.001; compared to the model group, **P*<0.05. | | | | |

Mice were sensitized with OVA+$Al(OH)_3$ for 3 weeks, followed by continuous stimulation with 5% OVA for 3 days. There was a significant increase in the number of inflammatory cells in bronchoalveolar lavage fluid (P<0.001 or P<0.01). Compared with the model group, pre-administration of the compound described in Example 3 before stimulation could significantly reduce the number of inflammatory cells (white blood cells, lymphocytes).

**Test example 6: Four-Week Repeat-Dose Toxicity Study in Rats**

6.1 Materials:

Animals: SD rats, SPF grade, with an equal number of males and females, weighing 200±20 g;

Test sample: Example 3 compound cream (2%), referencing the preparation of commercially available Benvitimod cream;

Positive control: BMS-986165;

Reagents: urethane, reagent for hematology analyzer, reagent for full-automatic biochemical analyzer, reagent for full-automatic coagulation analyzer, and reagent for electrolyte analyzer;

Instruments: electronic balance, hematology analyzer, full-automatic biochemical analyzer, full-automatic coagulation analyzer, and electrolyte analyzer.

6.2 Group Assignment and Dosing Regimen:

Grouping: the animals were randomly divided into three groups based on body weight: blank control group, Example 3 compound group, and BMS-986165 group. Each group contained 10 animals, with an equal number of males

and females.

Administration regimen: the Example 3 compound group received twice-daily topical application on the skin, while the BMS-986165 group received twice-daily gavage administration. The treatment lasted for four consecutive weeks (28 days). The specific administration regimen was shown in the following table:

| Group | Treatment | Administration dosage/frequen cy | Administr ation mode | Dose (mg/kg/d) |
|---|---|---|---|---|
| Blank control group | Blank matrix | 500 mg/bid | ext | - |
| Example 3 compound group | Compound of Example 3 | 500 mg/bid | ext | 100 |
| BMS-986165 group | BMS-986165 | 2 mL/bid | po | 50 |

6.3 Parameters Monitored:

Daily clinical observation and body weight measurement;

After the last administration, rats were fasted for more than 16h, anesthetized on the next day, and blood was collected from abdominal aorta. Tests such as hematology (EDTA-2K anticoagulation), blood biochemistry (serum), coagulation function (separated plasma with sodium citrate anticoagulation) and the like were respectively carried out;

After euthanization of the animals, the tissue organs were dissected and observed in general, and organ weights and coefficients (heart, liver, spleen, kidney, thymus) were determined;

The skin and lung tissues of the administration site were taken for HE staining to observe the changes in epidermal thickness, dermal inflammatory cells and pulmonary lesions.

6.4 Results:

| Parameter | | Blank control group | Example 3 compound group | BMS-986165 group |
|---|---|---|---|---|
| | | - | 100mg/kg | 50mg/kg |
| Hematology | WBC ($\times 10^9$/L) | 9.0±1.0 | 8.4±3.8 | 5.4±1.2** |
| | Lymph ($\times 10^9$/L) | 5.7±1.6 | 6.4±2.7 | 3.6±0.8* |
| | Mono ($\times 10^9$/L) | 0.3±0.1 | 0.2±0.1 | 0.2±0.0* |
| | Gran ($\times 10^9$/L) | 2.9±1.3 | 1.7±1.0 | 1.6±0.6 |
| | PLT ($\times 10^9$/L) | 1246.3±156.6 | 1001.8±408.1 | 433.8±474.5* |
| Coagulation | APTT (s) | 17.7±1.8 | 19.8±1.9 | 31.1±3.4** |
| Blood biochemistry | TB (umol/L) | 3.3±0.4 | 2.9±0.7 | 5.1±1.1* |
| Notes: compared to the blank control group: *$P$<0.05, **$P$<0.01. | | | | |

WBC: white blood cells; Lymph: lymphocytes; Mono: monocytes; Gran: granulocytes; PLT: platelets; APTT: activated partial thromboplastin time; TB: total bilirubin.

The hematological results showed that BMS-986165 could significantly reduce the number of white blood cells (P<0.01) and platelets (P<0.05) in the rats, while the compound of Example 3 had no significant effect on the number of white blood cells (P>0.05);

The coagulation results showed that BMS-986165 could significantly prolong APTT (P<0.01), while the compound of Example 3 had no significant effect on APTT (P>0.05);

The blood biochemistry results showed that BMS-986165 could significantly increase the level of total bilirubin (TB) in serum (P<0.05), while the compound of Example 3 had no significant effect on total bilirubin (P>0.05).

In addition, the compound of Example 3 showed no significant effects on the general state, body weight, organ coefficient, skin and Pulmonary pathology, and other hematological, coagulation, and blood biochemical indicators, indicating a lower potential for toxicity.

In addition, the compounds of the present invention showed a lower potential for toxicity in skin irritation tests, skin sensitization tests, and oral acute toxicity tests.

Finally, it should be noted that the above examples are only used to illustrate the technical solution of the present invention, and not to limit it. Although the present invention has been described in detail with reference to the foregoing embodiments, those of ordinary skill in the art should understand that they can still modify the technical solutions described in the foregoing embodiments, or perform equivalent substitutions for some or all of the technical features. These modifications or substitutions do not make the essence of the corresponding technical solutions deviate from the scope of the technical solutions of the embodiments of the present invention.

## Claims

1. A novel aromatic ring derivative containing an amide substitution or a pharmaceutically acceptable salt thereof, **characterized in that**, the compound is expressed by formula I:

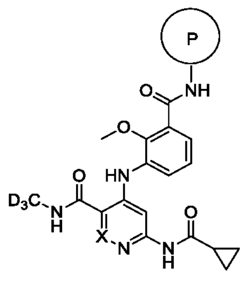

(I)

wherein, in the compound of formula I, X and P are selected independently from each other, and:

X is selected from CH, N;
P is selected from C1-C6 alkyl, substituted or unsubstituted C6-C10 aryl, substituted or unsubstituted five-membered heteroaryl, substituted or unsubstituted six-membered heteroaryl, wherein the substituent is selected from one or more of: C1-C6 alkyl, C3-C6 cycloalkyl, halogen, C1-C6 alkoxy, C1-C6 haloalkyl, and -CN;
when X is N, P is as defined above; or
when X is CH, P is as defined above.

2. The compound of formula I or a pharmaceutically acceptable salt thereof as claimed in claim 1, **characterized in that**, in the compound of formula I, X and P are selected independently from each other, and:

X is selected from CH, N;
P is selected from C1-C4 alkyl, substituted or unsubstituted phenyl, substituted or unsubstituted five-membered heteroaryl containing 2 to 3 N, substituted or unsubstituted six-membered heteroaryl containing 1 to 2 N, wherein the substituent is selected from 1 or 2 of: C1-C4 alkyl, C3-C6 cycloalkyl, halogen, C1-C4 alkoxy, C1-C4 haloalkyl, and -CN;
when X is N, P is as defined above; or
when X is CH, P is as defined above.

3. The compound of formula I or a pharmaceutically acceptable salt thereof as claimed in claim 1, **characterized in that**, in the compound of formula I, X and P are selected independently from each other, and:

X is selected from CH, N;

P is selected from tert-butyl, substituted or unsubstituted phenyl, substituted or unsubstituted five-membered heteroaryl selected from

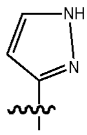 , 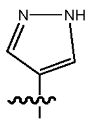 , 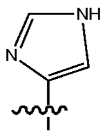 and 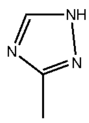 ,

and substituted or unsubstituted six-membered heteroaryl selected from

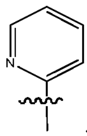 , 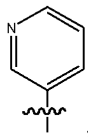 , 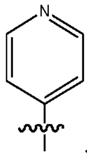 ,

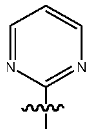 and 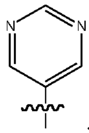 ,

wherein the substituent is selected from 1 or 2 of: methyl, ethyl, isopropyl, tert-butyl, cyclopropyl, methoxy, cyano, fluoro and trifluoromethyl;
when X is N, P is as defined above; or
when X is CH, P is as defined above.

**4.** The compound of formula I or a pharmaceutically acceptable salt thereof as claimed in claim 1, **characterized in that**, in the compound of formula I, X and P are selected independently from each other, and:

X is selected from CH, N;
P is selected from tert-butyl, substituted or unsubstituted phenyl, substituted or unsubstituted five-membered heteroaryl selected from

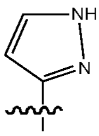 , 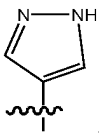 , 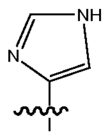 and 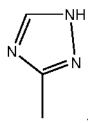 ,

and substituted or unsubstituted six-membered heteroaryl selected from

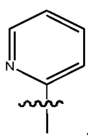 , 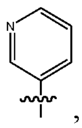 , 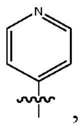 ,

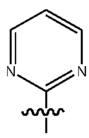 and 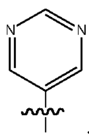 ,

for the above phenyl, the substituent is selected from 1 or 2 of: methyl, ethyl, isopropyl, tert-butyl, cyclopropyl, methoxy, cyano, fluoro and trifluoromethyl;
for the above five-membered heteroaryl, the substituent is methyl;
for the above six-membered heteroaryl, the substituent is selected from methyl or fluoro;

when X is N, P is as defined above; or
when X is CH, P is as defined above.

**5.** The compound of formula I or a pharmaceutically acceptable salt thereof as claimed in claim 1, **characterized in that**, in the compound of formula I, X and P are selected independently from each other, and:

X is selected from CH, N;
P is selected from tert-butyl,

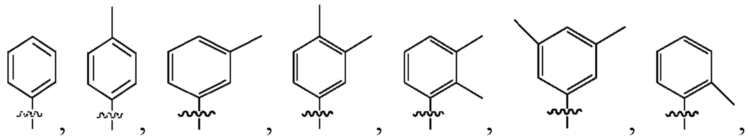

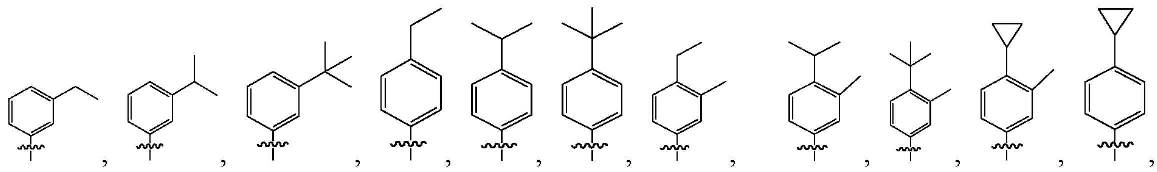

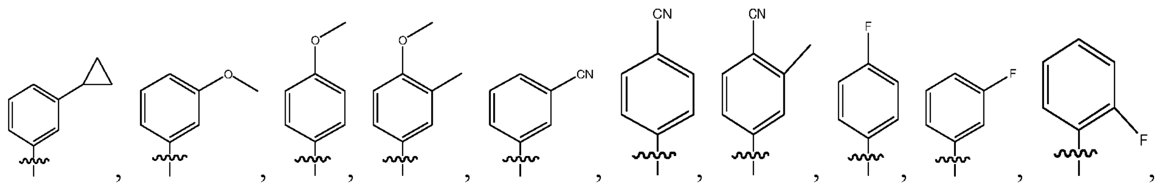

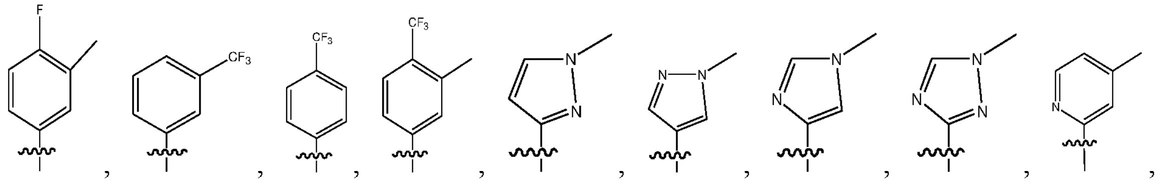

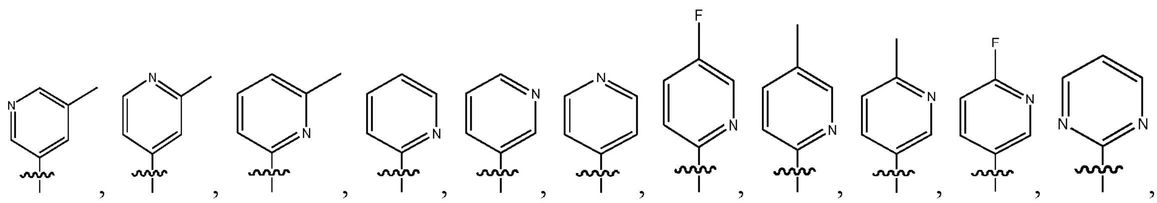

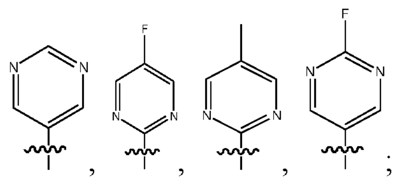

when X is N, P is as defined above; or
when X is CH, P is as defined above.

**6.** The compound of formula I or a pharmaceutically acceptable salt thereof as claimed in claim 1, **characterized in that**, in the compound of formula I, X and P are selected independently from each other, and:

X is selected from CH or N;

P is selected from substituted or unsubstituted phenyl, substituted or unsubstituted five-membered heteroaryl

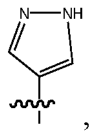 ,

substituted or unsubstituted six-membered heteroaryl

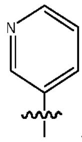 ,

for the above phenyl, the substituent is selected from 1 or 2 of: methyl, fluorine;
for the above five-membered heteroaryl, the substituent is methyl;
for the above six-membered heteroaryl, the substituent is methyl;
preferably, P is selected from:

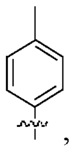 , 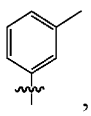 , 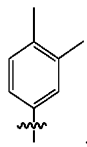 , 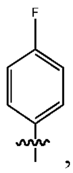 , 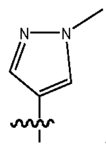 , 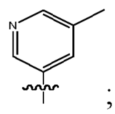 ;

when X is N, P is as defined above; or
when X is CH, P is as defined above.

7. The novel aromatic ring derivative containing an amide substitution or a pharmaceutically acceptable salt thereof as claimed in any of claims 1-5, **characterized in that**, the compound of formula I is selected from:

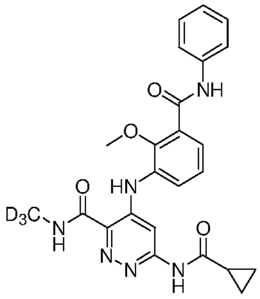
1

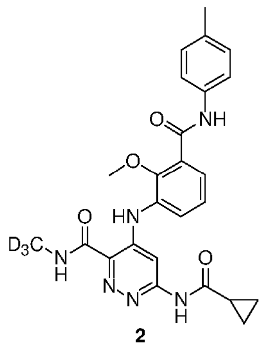
2

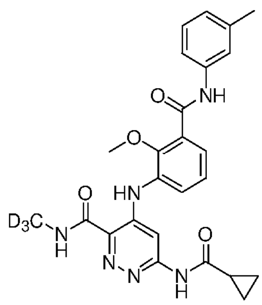
3

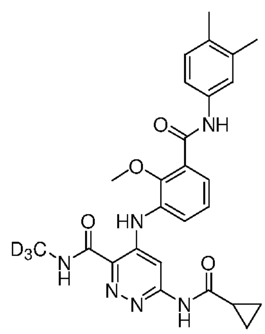
4

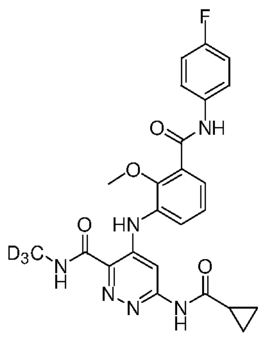
5

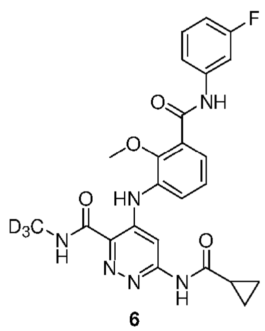
6

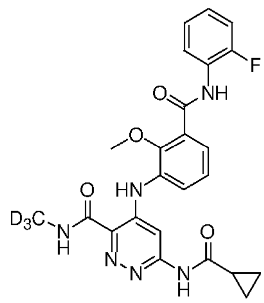
7

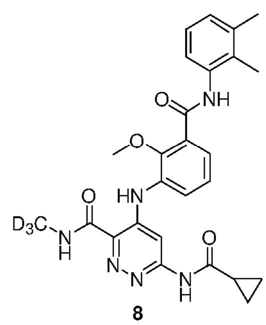
8

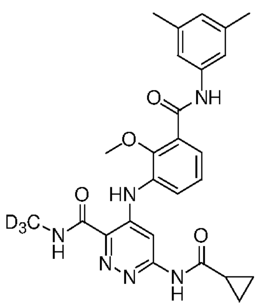
**9**

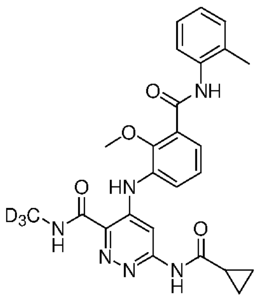
**10**

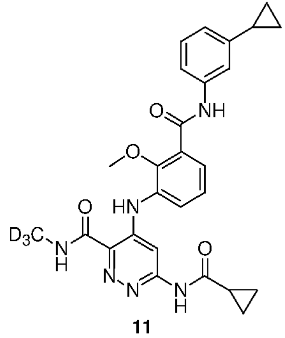
**11**

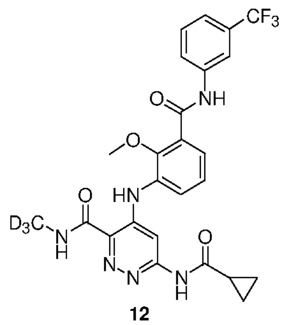
**12**

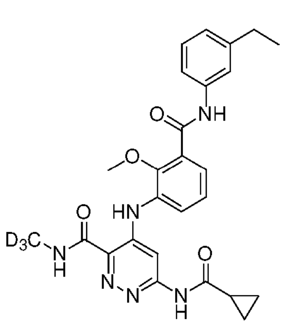
**13**

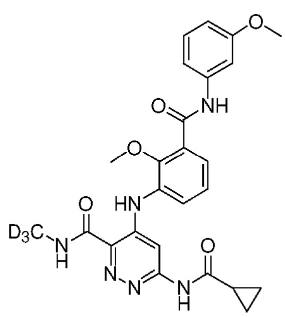
**14**

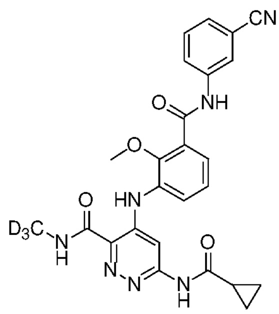
**15**

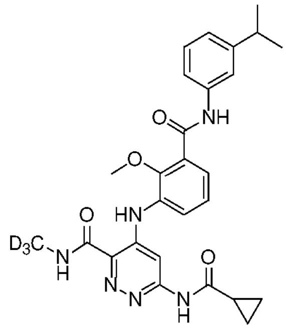
**16**

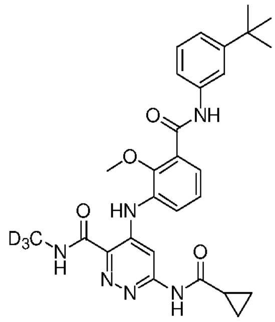
**17**

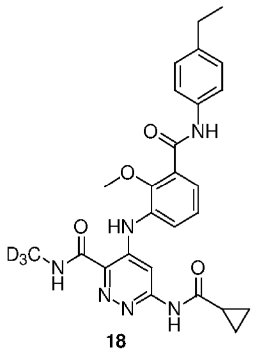
**18**

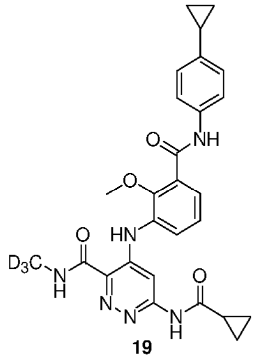
**19**

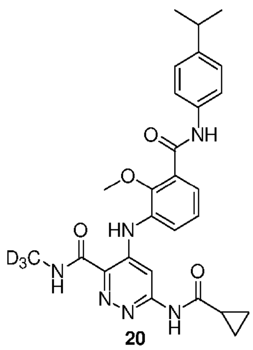
**20**

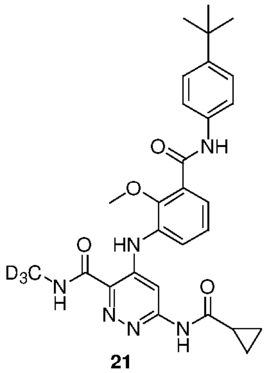
**21**

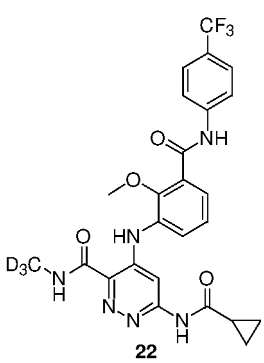
**22**

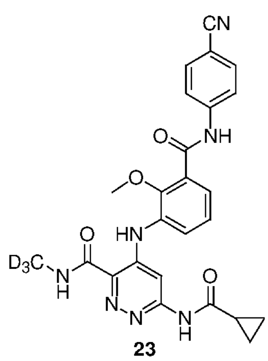
**23**

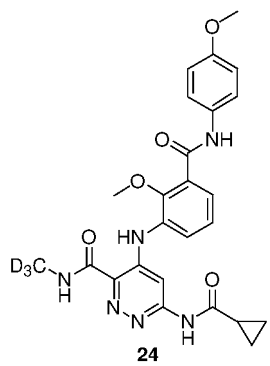
**24**

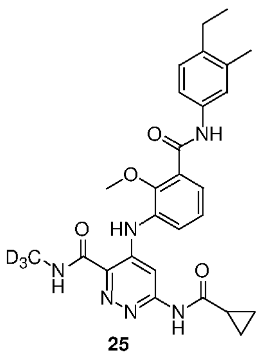
25

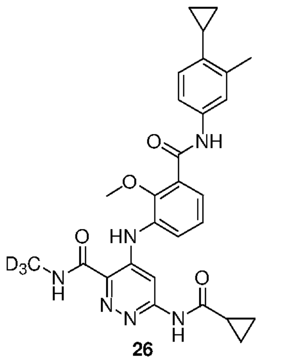
26

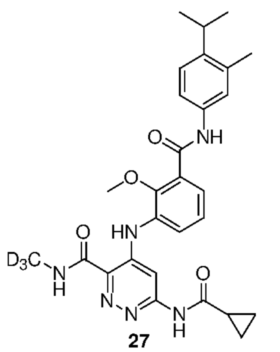
27

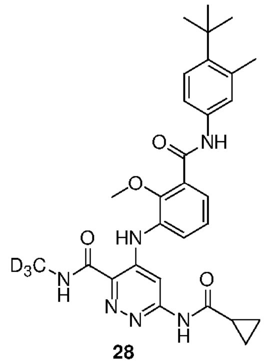
28

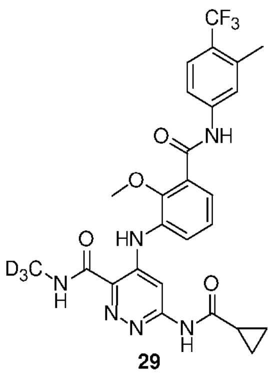
29

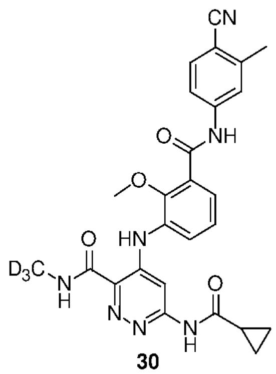
30

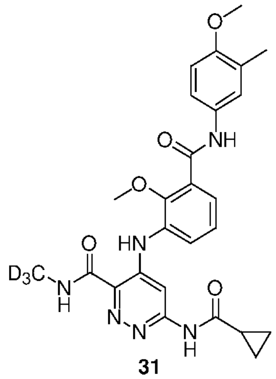
31

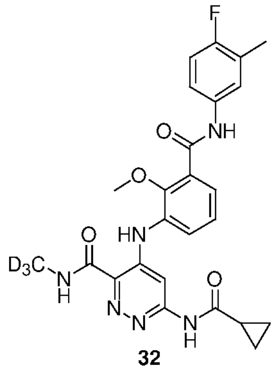
32

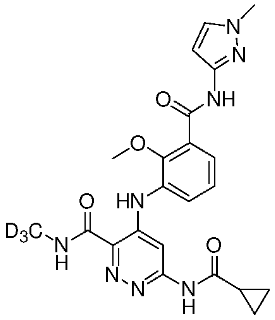
33

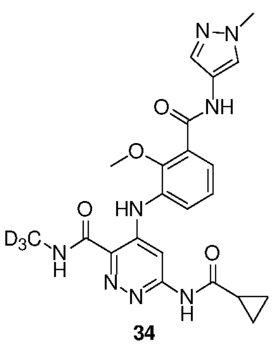
34

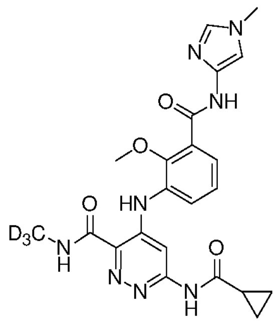
35

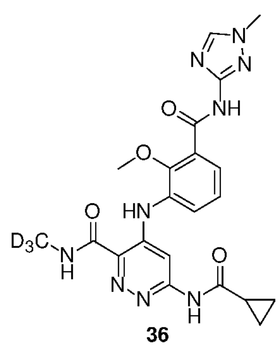
36

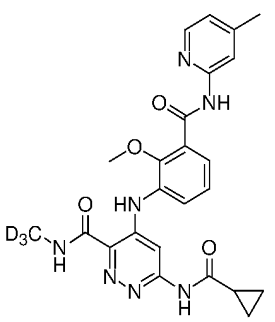
37

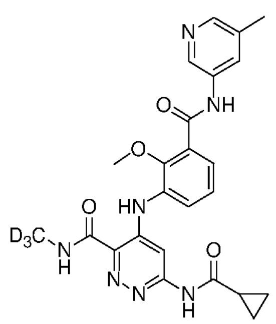
38

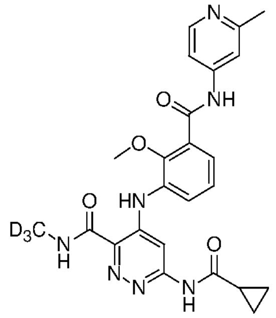
39

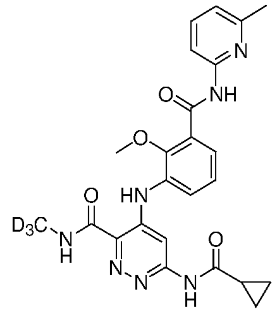
40

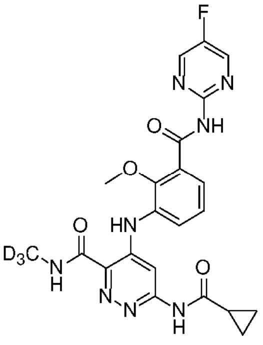

**41**

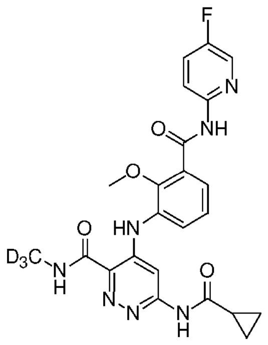

**42**

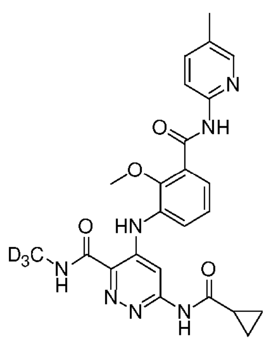

**43**

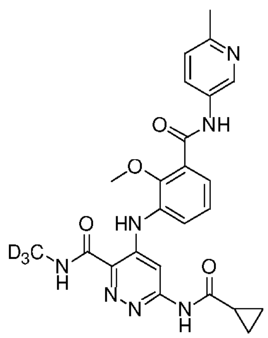

**44**

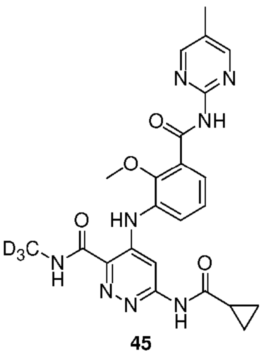

**45**

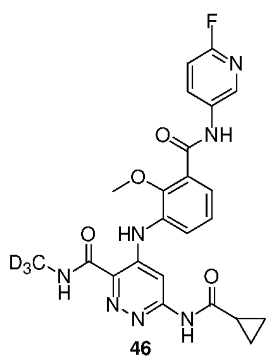

**46**

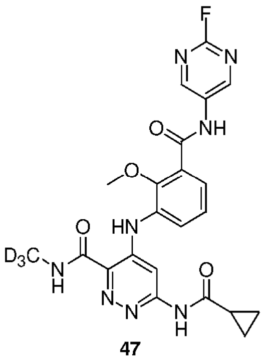

**47**

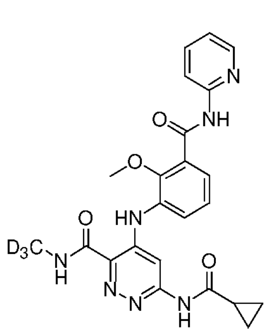

**48**

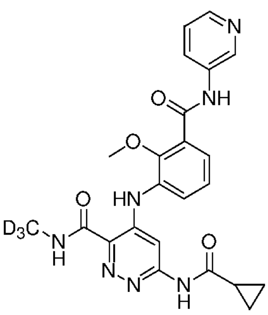

**49**

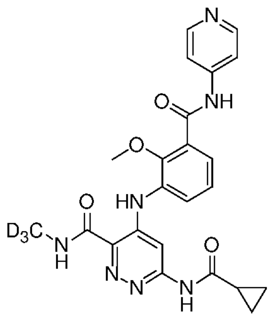

**50**

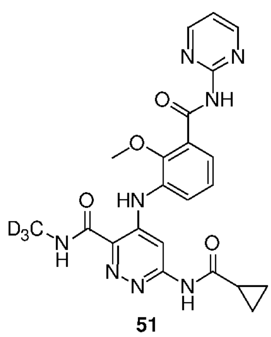

**51**

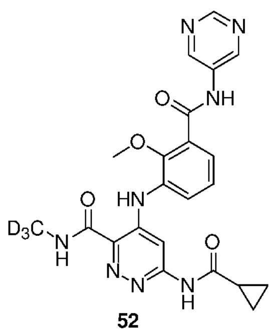

**52**

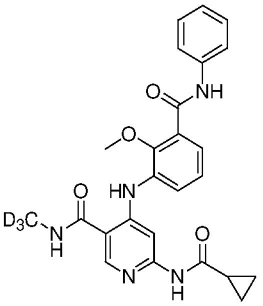

**53**

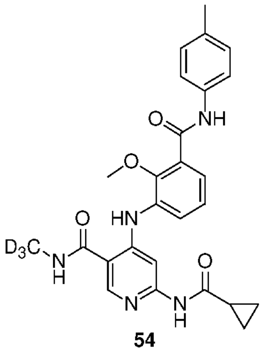

**54**

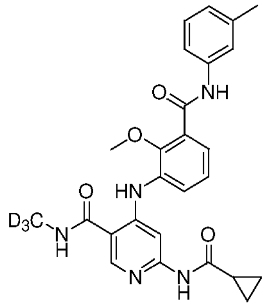

**55**

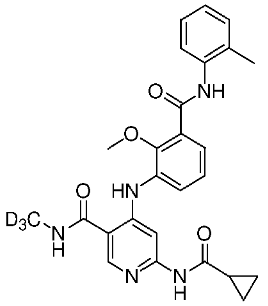

**56**

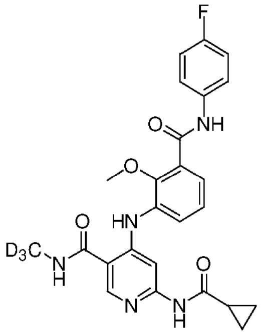
57

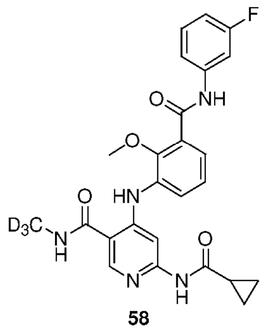
58

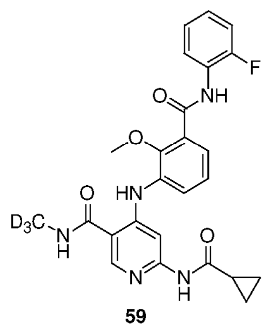
59

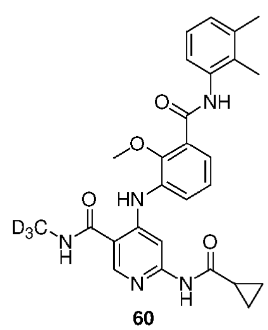
60

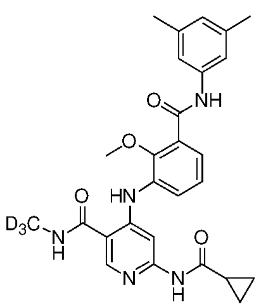
61

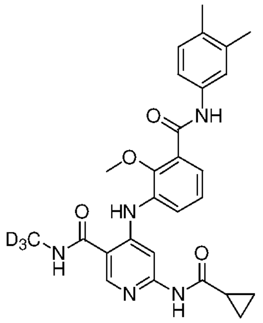
62

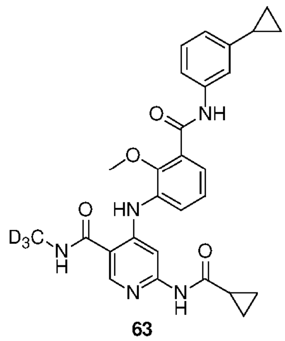
63

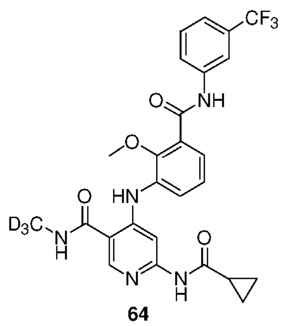
64

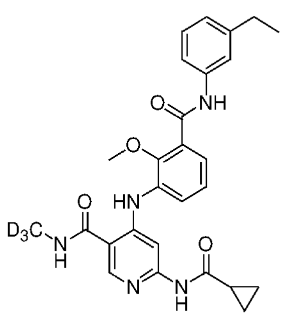
65

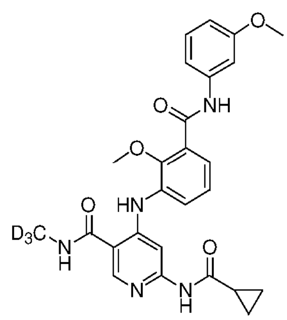
66

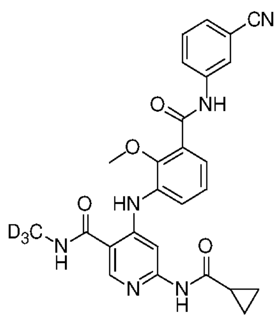
67

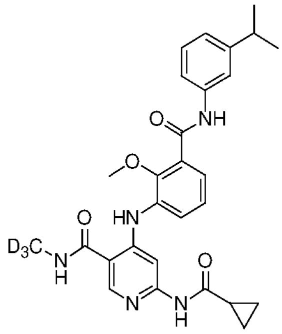
68

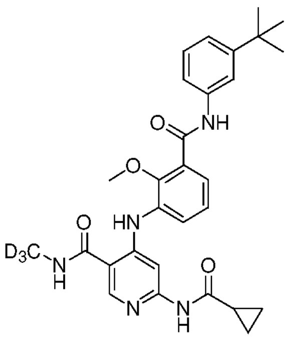
69

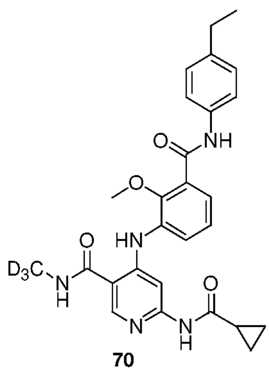
70

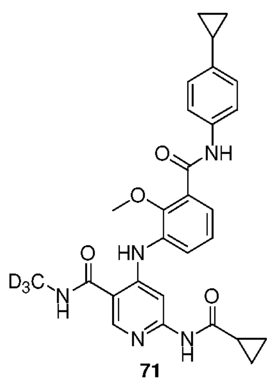
71

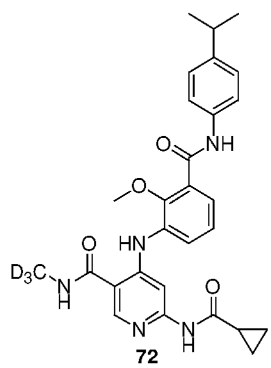
72

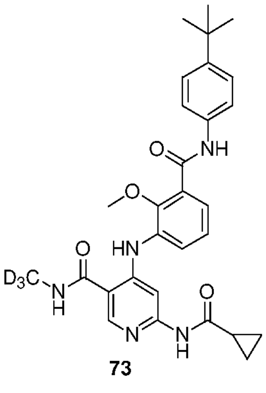

73

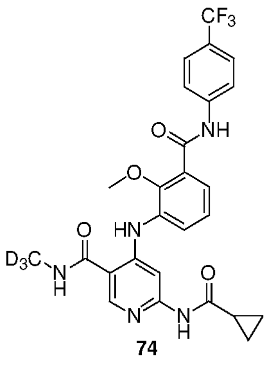

74

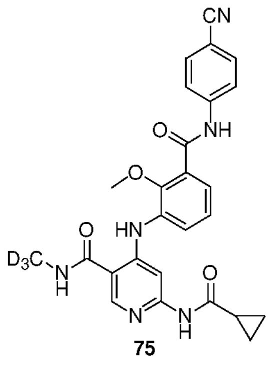

75

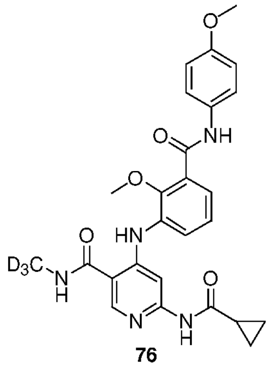

76

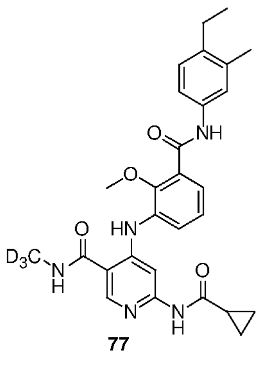

77

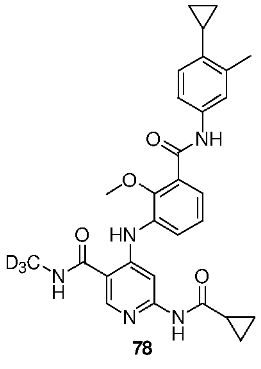

78

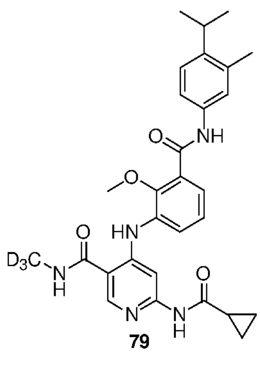

79

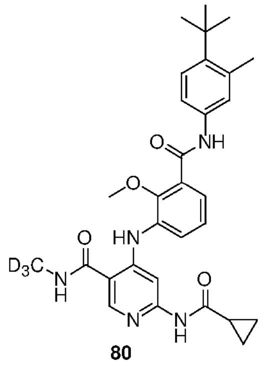

80

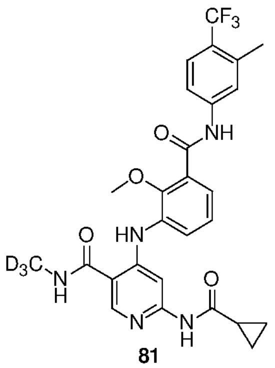

81

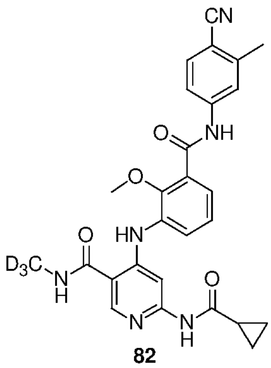

82

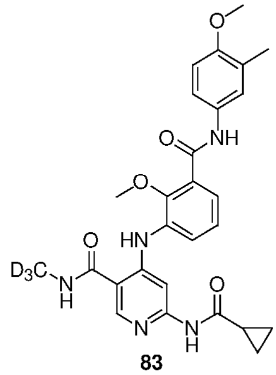

83

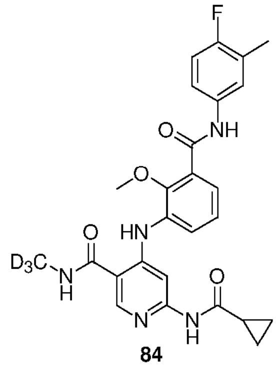

84

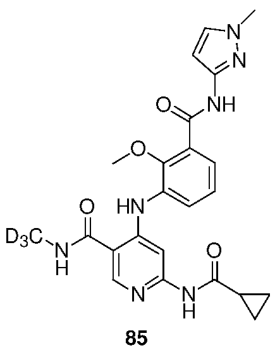

85

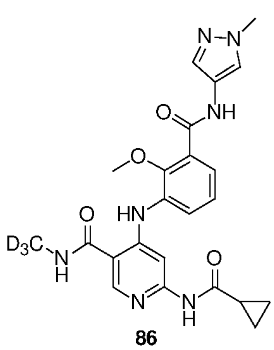

86

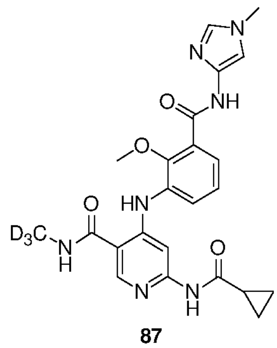

87

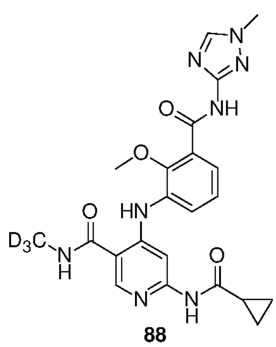

88

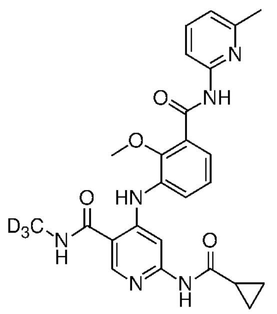

89

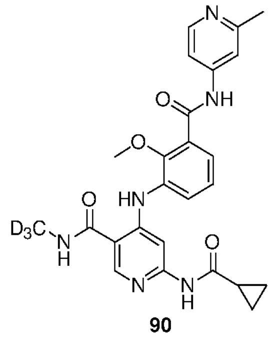

90

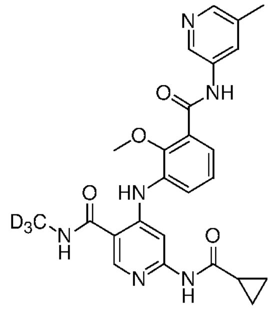

91

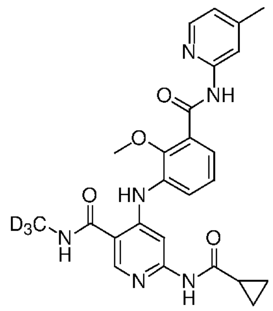

92

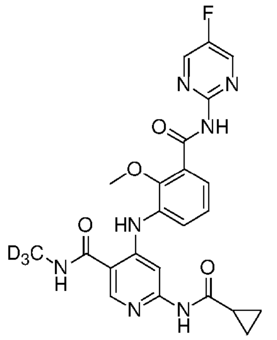

93

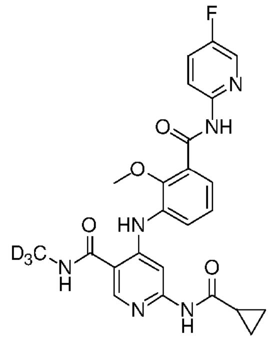

94

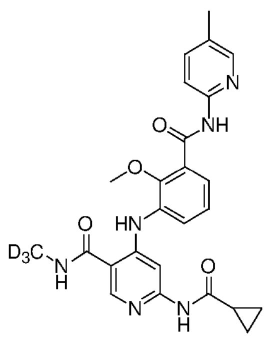

95

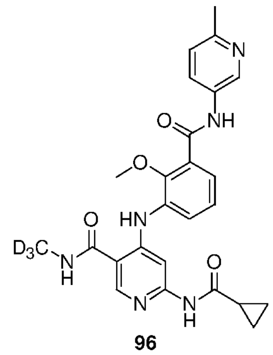

96

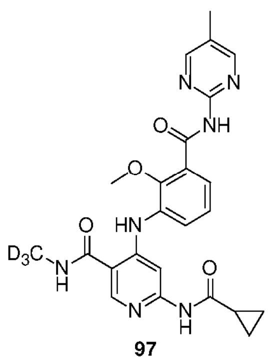

97

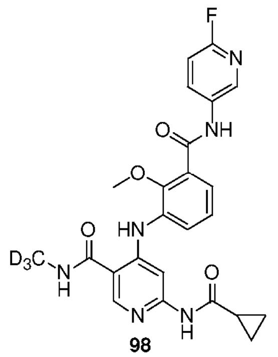

98

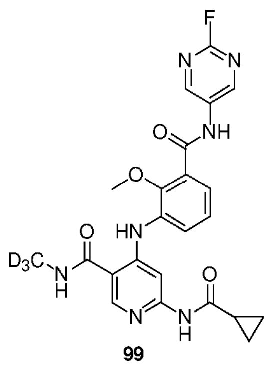

99

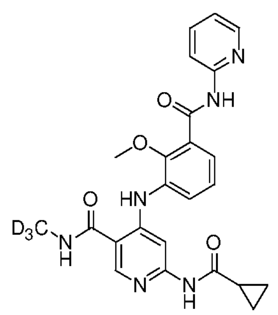

100

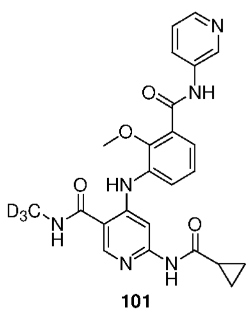

101

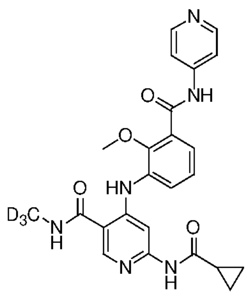

102

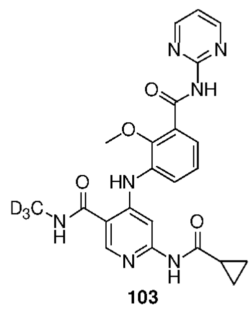

103

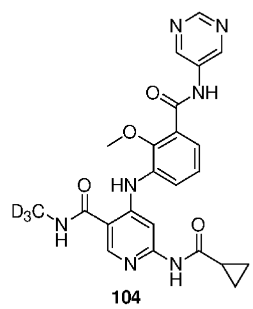

104

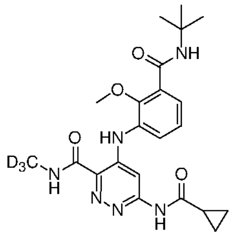

105

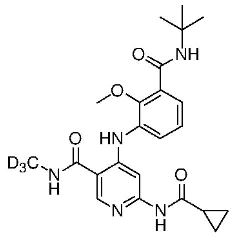

106

.

8. The novel aromatic ring derivative containing an amide substitution or a pharmaceutically acceptable salt thereof as claimed in claim 7, **characterized in that**, the compound of formula I is selected from: compound 2, compound 3, compound 4, compound 5, compound 34, compound 38, compound 54, compound 55, compound 57, compound 62, compound 86, and compound 91.

9. A pharmaceutical composition, **characterized in that**, comprising the novel aromatic ring derivative containing an amide substitution or a pharmaceutically acceptable salt thereof as claimed in any of claims 1-8 and optionally one or more pharmaceutically acceptable carriers, diluents, excipients or adjuvants.

10. Use of the novel aromatic ring derivative containing an amide substitution or a pharmaceutically acceptable salt thereof as claimed in any of claims 1-8 or the pharmaceutical composition as claimed in claim 9 in the manufacture of a medicament for the treatment of TYK 2-mediated related diseases.

11. The use as claimed in claim 10, **characterized in that**, the disease is selected from inflammatory or autoimmune diseases.

12. The use as claimed in claim 10 or 11, **characterized in that**, the disease is selected from psoriasis, psoriatic arthritis, dermatitis, lupus erythematosus, inflammatory bowel disease, hidradenitis suppurativa, rheumatoid arthritis or uveitis.

## INTERNATIONAL SEARCH REPORT

International application No.
**PCT/CN2023/140951**

**A. CLASSIFICATION OF SUBJECT MATTER**

C07D237/24(2006.01)i; C07D403/12(2006.01)i; C07D401/12(2006.01)i; C07D213/82(2006.01)i; C07B59/00(2006.01)i; A61K31/50(2006.01)i; A61K31/501(2006.01)i; A61K31/506(2006.01)i; A61K31/44(2006.01)i; A61K31/4439(2006.01)i; A61K31/444(2006.01)i; A61P17/06(2006.01)i; A61P19/02(2006.01)i; A61P17/00(2006.01)i; A61P1/00(2006.01)i; A61P29/00(2006.01)i; A61P27/02(2006.01)i

According to International Patent Classification (IPC) or to both national classification and IPC

**B. FIELDS SEARCHED**

Minimum documentation searched (classification system followed by classification symbols)

IPC: C07D C07B A61K A61P

Documentation searched other than minimum documentation to the extent that such documents are included in the fields searched

Electronic data base consulted during the international search (name of data base and, where practicable, search terms used)

CNABS, CNTXT, CNKI, 万方数据库, WANFANG DATABASE, 百度学术, BAIDU SCHOLAR, VEN, ENTXT, STN: 津药生物科技（天津）有限公司; 环丙甲酰胺, 环丙烷酰胺基, (甲基-d3)哒嗪-3-甲酰胺, 自身免疫, 炎症, TYK2, JAK, autoimmune, inflammation, cyclopropanecarboxamido, (methyl-d3)pyridazine-3-carboxamide.

**C. DOCUMENTS CONSIDERED TO BE RELEVANT**

| Category* | Citation of document, with indication, where appropriate, of the relevant passages | Relevant to claim No. |
|---|---|---|
| X | CN 111484480 A (SHANGHAI HANSOH BIOMEDICAL CO., LTD. et al.) 04 August 2020 (2020-08-04)<br>description, pages 2 and 11-17, and description, page 39, embodiment 39 | 1-12 |
| A | LIU, Fei et al. "Novel TYK2 Inhibitors with an N-(Methyl-d3)pyridazine-3-carboxamide Skeleton for the Treatment of Autoimmune Diseases"<br>*ACS Medicinal Chemistry Letters,* Vol. 13, No. 11, 06 October 2022 (2022-10-06), 1730-1738 ISSN: 1948-5875,<br>pages 1730-1738 | 1-12 |
| A | CN 114057651 A (CHIA TAI TIANQING PHARMACEUTICAL GROUP CO., LTD.) 18 February 2022 (2022-02-18)<br>entire document | 1-12 |
| A | WO 2022121868 A1 (CHIA TAI TIANQING PHARMACEUTICAL GROUP CO., LTD.) 16 June 2022 (2022-06-16)<br>entire document | 1-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 March 2024** | **23 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No. PCT/CN2023/140951

C. DOCUMENTS CONSIDERED TO BE RELEVANT

| Category* | Citation of document, with indication, where appropriate, of the relevant passages | Relevant to claim No. |
|---|---|---|
| A | CN 114907326 A (CHIA TAI TIANQING PHARMACEUTICAL GROUP CO., LTD.) 16 August 2022 (2022-08-16) entire document | 1-12 |
| A | WO 2022233286 A1 (SHANGHAI ZHEYE BIOTECHNOLOGY CO., LTD.) 10 November 2022 (2022-11-10) entire document | 1-12 |

Form PCT/ISA/210 (second sheet) (July 2022)

**INTERNATIONAL SEARCH REPORT**
**Information on patent family members**

International application No.
**PCT/CN2023/140951**

| Patent document cited in search report | | | Publication date (day/month/year) | Patent family member(s) | | | Publication date (day/month/year) |
|---|---|---|---|---|---|---|---|
| CN | 111484480 | A | 04 August 2020 | CN | 111484480 | A | 11 August 2023 |
| CN | 114057651 | A | 18 February 2022 | None | | | |
| WO | 2022121868 | A1 | 16 June 2022 | CN | 116547276 | A | 04 August 2023 |
| CN | 114907326 | A | 16 August 2022 | None | | | |
| WO | 2022233286 | A1 | 10 November 2022 | AU | 2022268464 | A1 | 21 December 2023 |
| | | | | KR | 20240016974 | A | 06 February 2024 |
| | | | | CN | 117136058 | A | 28 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201380069692